(19) Europäisches Patentamt
European Patent Office
Office européen des brevets

(11) **EP 4 342 496 A1**

(12) **EUROPEAN PATENT APPLICATION**
published in accordance with Art. 153(4) EPC


(43) Date of publication:
**27.03.2024 Bulletin 2024/13**

(21) Application number: **22804753.6**

(22) Date of filing: **19.05.2022**

(51) International Patent Classification (IPC):
***A61K 47/16*** (2006.01) ***A61K 9/10*** (2006.01)
***A61K 9/127*** (2006.01) ***A61K 31/7105*** (2006.01)
***A61K 31/713*** (2006.01) ***A61K 47/02*** (2006.01)
***A61K 47/24*** (2006.01) ***A61K 47/28*** (2006.01)
***A61K 47/44*** (2017.01) ***A61K 48/00*** (2006.01)
***A61P 35/00*** (2006.01) ***A61P 37/04*** (2006.01)

(52) Cooperative Patent Classification (CPC):
**A61K 9/10; A61K 9/127; A61K 31/23;**
**A61K 31/5375; A61K 31/575; A61K 31/661;**
**A61K 31/7105; A61K 31/713; A61K 31/77;**
**A61K 47/02; A61K 47/10; A61K 47/16;**
**A61K 47/24; A61K 47/28; A61K 47/44;** (Cont.)

(86) International application number:
**PCT/JP2022/020849**

(87) International publication number:
**WO 2022/244844 (24.11.2022 Gazette 2022/47)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **21.05.2021 JP 2021085985**



(71) Applicant: **National University Corporation Hokkaido University**
**Sapporo-shi, Hokkaido 060-0808 (JP)**

(72) Inventors:
- **SATO Yusuke**
  **Sapporo-shi, Hokkaido 060-0808 (JP)**
- **SASAKI Kosuke**
  **Sapporo-shi, Hokkaido 060-0808 (JP)**
- **HARASHIMA Hideyoshi**
  **Sapporo-shi, Hokkaido 060-0808 (JP)**

(74) Representative: **Forstmeyer, Dietmar et al**
**Boeters & Lieck**
**Oberanger 32**
**80331 München (DE)**


(54) **LIPID NANOPARTICLES**


(57) The present invention provides lipid nanoparticles that contain a pH-sensitive cationic lipid and a polyalkylene glycol-modified lipid. The pH-sensitive cationic lipid fraction of the total lipids that constitute the lipid nanoparticles is 10-25 mol%, the polyalkylene glycol-modified lipid fraction of the total lipids that constitute the lipid nanoparticles is 0.5-1.75 mol%, and the number average particle size of the lipid nanoparticles is at least 150 nm.


[FIG. 18]

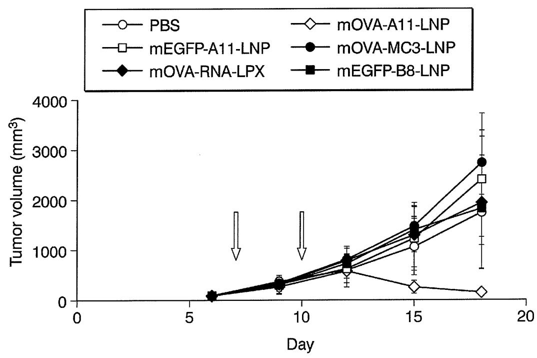


EP 4 342 496 A1

(52) Cooperative Patent Classification (CPC): (Cont.)
**A61K 48/00; A61P 35/00; A61P 37/04**

**Description**

TECHNICAL FIELD

The present invention relates to a lipid nanoparticle useful as a gene delivering carrier that is capable of a highly efficient delivery to splenic dendritic cells.

BACKGROUND ART

One of the modalities which has been gathering attention for clinical application as a gene therapeutic includes an mRNA medicine. The mRNA medicine is a next-generation pharmaceutical agent which is an artificially synthesized mRNA, a biogenic substance, to be administrated into an organism to express a protein that is desired for treating a disease, etc. An mRNA has no risk of inserting a mutation into the genome, and its sequence can be designed as soon as the gene is identified, and theoretically it is capable to express any protein. Accordingly, it has been gathering attention as a pharmaceutical molecule that can be applied to a wide range of disease areas. Thus, the mRNA, in particular a mRNA as a vaccine, is considered to greatly contribute to an individualized cancer therapy and to launching a quick response to a viral mutation and a pandemic in the area of infections (Non-Patent Literature 1). In the development of vaccines against SARS-CoV-2, Moderna, Inc. (United States of America) shipped the first lot of the investigational drug (a mRNA-loading lipid nanoparticle preparation) only 42 days after the public disclosure of the viral genome DNA sequence, and this fact demonstrated that the mRNA vaccine is a technique that is capable of launching a quick response for an infection control measure.

It is generally said that a vaccine needs an action of immunoactivation. On the other hand, a single-strand RNA is principally recognized by Toll-like receptor 7/8 (TLR7/8) localized in endosome and induces Interferon Type I response. That is, the mRNA possesses a strong immunogenicity via the innate immune mechanism. Therefore, in a mRNA vaccine, mRNA plays roles both in expressing target protein and in immunoactivating action (Non-Patent Literatures 2 and 3).

Lipid nanoparticles (LNPs) are utilized as carriers for encapsulating nucleic acids such as mRNAs or siRNAs (small interfering RNAs) and delivering them to target cells. For instance, a lipid nanoparticle comprising as a constituent lipid a pH-sensitive cationic lipid, which is electrically neutral at physiological pH and turns cationic under weakly acidic pH environment, is reported as a lipid nanoparticle that can be used as a carrier for efficiently delivering a nucleic acid to a target cell (Patent Literature 1). A lipid nanoparticle which is positively charged in blood will cause nonspecific interaction with plasma proteins and will quickly be eliminated from the blood by the reticuloendothelial system. On the contrary, the lipid nanoparticle comprising the pH-sensitive cationic lipid as the constituent lipid is not charged under neutral condition in blood, but is positively charged under weakly acidic condition inside the endosome, and can thereby efficiently escape from the endosome.

As an mRNA vaccine for systemic administration, an RNA-lipoplex (RNA-LPX) has been reported from BioNTech SE. (Non-Patent Literature 2). The RNA-LPX has a lipid composition comprising 1,2-di-O-octadecenyl-3-trimethylammonium propane (DOTMA) and 1,2-dioleoyl-sn-glycero-3-phosphoethanolamine (DOPE) at a molar ratio of 1 : 1. It is prepared using a liposome prepared by a simple hydration method or ethanol-dilution method, combining the liposome with mRNA such that the N/P ratio which is the ratio of the number of quaternary ammonium groups contained in DOTMA to the number of phosphate groups in mRNA is 1.3/2. The RNA-LPX exhibits very high specificity for spleen as indicated by gene expression, and exhibits a high gene expression in splenic dendritic cell, in particular. The RNA-LPX is, at present (February, 2021), undergoing Phase I clinical trials for applications to melanoma, prostate cancer, human papillomavirus type 16-positive head and neck cancer, triple-negative breast cancer, and ovarian cancer (Non-Patent Literature 3). In addition, Tozinameran is an LNP-type preparation (Non-Patent Literature 4), which was the world's first mRNA pharmaceutical agent granted Emergency Use Authorization by United States Food and Drug Administration (FDA) on December 11, 2020, as a prophylactic vaccine for COVID-19 (Corona virus disease-19).

CITATION LIST

PATENT LITERATURES

Patent Literature 1: WO 2018/230710
Patent Literature 2: WO 2018/190423

NON-PATENT REFERENCES

Non-Patent Literature 1: Pardi et al., NATURE REVIEWS DRUG DISCOVERY, 2018, vol. 17, p.261-279.
Non-Patent Literature 2: Kranz et al., Nature, 2016, vol.534, p.396-401.
Non-Patent Literature 3: Sahin et al., Nature, 2020, vol.585, p.107-112.
Non-Patent Literature 4: Polack et al., The New ENGLAND JOURNAL of MEDICINE, 2020, vol. 383(27), p.2603-2615.
Non-Patent Literature 5: Sato et al., Journal of Controlled Release, 2019, vol.295, p.140-152.
Non-Patent Literature 6: Stroock et al., Science, 2002, vol.295, p.647-651.
Non-Patent Literature 7: Hajj et al., Nano Letters, 2020, vol.20, p. 5167-5175.

SUMMARY OF INVENTION

PROBLEMS TO BE SOLVED BY INVENTION

The RNA-LPX has preceded as mRNA cancer vaccine for systemic administration. However, there still are few reports on systems that enables an efficient delivery of nucleic acid to cells other than hepatic parenchymal cells, including immunocytes, and also there is room for improvement in efficiency.
An object of the present invention is to provide a lipid nanoparticle used as a gene delivering carrier that is capable of highly efficient delivery to splenic dendritic cells.

MEANS TO SOLVE THE PROBLEMS

The present inventors have found that the gene expression efficiency to splenic dendritic cell can be improved by including a pH-sensitive cationic lipid and a polyalkylene glycol-modified lipid as constituent lipids of a lipid nanoparticle for encapsulating a nucleic acid for gene expression such as siRNAs and adjusting the size of the lipid nanoparticle such that its number average particle diameter is equal to or greater than 150 nm, thus completed the present invention.
Accordingly, the present invention is to provide a lipid nanoparticle, etc. as follows.

[1] A lipid nanoparticle comprising a pH-sensitive cationic lipid and a polyalkylene glycol-modified lipid, wherein:

the proportion of the pH-sensitive cationic lipid content to the total amount of the lipids constituting the lipid nanoparticle is from 40 to 70 mol %; and
the proportion of the polyalkylene glycol-modified lipid content to the total amount of the lipids constituting the lipid nanoparticle is from 0.5 to 1.75 mol %;
and wherein the lipid nanoparticle has a number average particle diameter equal to or greater than 150 nm.

[2] The lipid nanoparticle according to [1], having a number average particle diameter equal to or smaller than 600 nm.

[3] The lipid nanoparticle according to [1] or [2], wherein the the pH-sensitive cationic lipid is represented by the following general formula (I-1):

[Chem. 1]

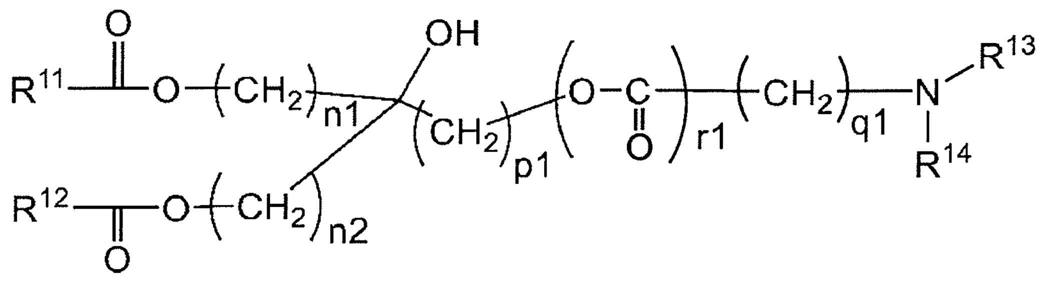

(I-1)

[wherein, in the formula (I-1), $R^{11}$ and $R^{12}$ are each independently a straight-chain $C_{10-14}$ alkyl group, a straight-

chain $C_{10-20}$ alkenyl group having one or two unsaturated bond(s), or -CH($R^{15}$)($R^{16}$) ($R^{15}$ and $R^{16}$ are each independently a straight-chain $C_{5-10}$ alkyl group); n1 and n2 each independently denotes an integer from 6 to 10; p1 and q1 denote integers that are equal to or greater than 0 and satisfy p1 + q1 = from 3 to 8; r1 denotes 0 or 1; $R^{13}$ and $R^{14}$ are each independently a straight-chain $C_{1-3}$ alkyl group or an aryl $C_{1-3}$ alkyl group, or $R^{13}$ and $R^{14}$ are joined together to form a pyrrolidine ring, a piperidine ring, a morpholine ring, or a piperazine ring in which a nitrogen atom may be substituted with a $C_{1-3}$ alkyl group].

[4] The lipid nanoparticle according to any one of [1] to [3], further comprising one or more selected from a group consisting of sterols and phospholipids.

[5] The lipid nanoparticle according to any one of [1] to [4], comprising a nucleic acid.

[6] The lipid nanoparticle according to [5], wherein the nucleic acid is an siRNA.

[7] The lipid nanoparticle according to [5], wherein the nucleic acid is an mRNA or a plasmid DNA.

[8] The lipid nanoparticle according to any one of [5] to [7], wherein the nucleic acid is a gene that is to be expressed in a splenic dendritic cell.

[9] A pharmaceutical composition, wherein the lipid nanoparticle according to any one of [1] to [8] is an active ingredient.

[10] The pharmaceutical composition according to [9] for use in cancer vaccine therapy.

[11] The pharmaceutical composition according to [9] for use in immunoactivation.

[12] A method of expressing an exogenous gene comprising administrating the lipid nanoparticle according to any one of [5] to [8] in which an exogenous gene of interest to be expressed in a splenic dendritic cell is encapsulated to a subject animal (excluding human) and expressing the exogenous gene in a splenic dendritic cell of the subject animal.

[13] A method for producing the lipid nanoparticle according to any one of [5] to [8] using a flow path structure, having

a forming step in which the lipid nanoparticle is formed in the flow path structure from a lipid solution in which all lipid components for constituting the lipid nanoparticle have been dissolved in ethanol and an aqueous solution comprising the nucleic acid; and
a dialysis step in which a solution comprising the lipid nanoparticle obtained in the forming step is dialyzed in a buffer solution at pH 6.8 to 7.6,

wherein:

the flow path structure forms one dilution flow path when a first introduction path for introducing a first fluid and a second introduction path for introducing a second fluid each independently has a specified length, and join together;
the dilution flow path has a flow path part that is two-dimensionally bent at least in a part thereof;
designating the axis direction of the dilution flow path upstream to the bent flow path part toward its extension direction to be the X direction, and designating the width direction of the dilution flow path that perpendicularly intersects this X direction to be the Y direction, and designating the width of the dilution flow path upstream to the bent flow path part to be $y_0$, the bent flow path part is formed by providing at least two or more structural elements with specified intervals $d_1$, $d_2$, ..., wherein the two or more structural elements protrude alternately from the both side faces of the dilution flow path opposing in the Y direction toward the center of the flow path, approximately in the Y direction (approximately in + Y direction and approximately in - Y direction), wherein the two or more structural elements have specified heights $h_1$, $h_2$, ... that are equal to or greater than 1/2 $y_0$ and smaller than 1 $y_0$, and have specified widths $x_1$, $x_2$, ..., in the X direction, and thereby define the width of the dilution flow path;
the lipid solution is introduced from the first introduction path and the aqueous solution comprising the nucleic acid is introduced from the second introduction path, respectively, and
the aqueous solution comprising the nucleic acid comprises the sodium chloride at a concentration equal to or

greater than 280 mM, and the aqueous solution comprising the nucleic acid is acidic.

[14] The method for producing a lipid nanoparticle according to [13], wherein the sodium chloride concentration in the aqueous solution comprising the nucleic acid is equal to or less than 500 mM.

[15] A kit for use in the method for producing a lipid nanoparticle according to [13] or [14], comprising

a dry matter of a lipid composition comprising all lipid components for constituting the lipid nanoparticle; and
a dry matter comprising sodium chloride for being dissolved in water for preparation of an aqueous solution in which the sodium chloride concentration is equal to or greater than 280 mM.

[16] The kit for use in the method for producing a lipid nanoparticle according to [15], further comprising one or more selected from the group consisting of ethanol, phosphate buffered saline and a nucleic acid.

## ADVANTAGEOUS EFFECTS OF INVENTION

The lipid nanoparticle according to the present invention is capable of expressing the encapsulated gene in splenic dendritic cells at a high level. Therefore, the lipid nanoparticle is useful as a splenic dendritic cell-specific gene delivering carrier which can be used in cancer vaccine therapy or gene therapy.

## BRIEF DESCRIPTION OF DRAWINGS

[FIG. 1] FIG. 1 is a diagram schematically showing the structure of a flow path structure in one embodiment that is used in the production of a lipid nanoparticle according to the present invention.
[FIG. 2] FIG. 2 is a diagram showing the reciprocal action profiles of factors which significantly influences on the size of lipid nanoparticle (ζ average particle diameter) by statistically analyzing the results of measuring physical properties of lipid nanoparticles of Examination Plots A-1 to A-14 in Example 1.
[FIG. 3] FIG. 3 is a diagram showing the results of measuring the Nluc expression level in the liver and spleen (per one milligram of protein) in the mice administered the lipid nanoparticles of Examination Plots A-1 to A-14 in Example 1.
[FIG. 4] FIG. 4 is a diagram showing the results of measuring the level of cellular uptake of the lipid nanoparticle by the dendritic cells, B cells, and macrophages in the spleen of the mice administered the lipid nanoparticles of Examination Plots A-1 to A-14 in Example 1.
[FIG. 5] FIG. 5 is a diagram showing the results of measuring the Nluc expression level in the dendritic cells, B cells, and macrophages in the spleen of the mice administered the lipid nanoparticles of Examination Plots A-1 to A-14 in Example 1.
[FIG. 6] FIG. 6 is a diagram showing the results of measuring the level of cellular uptake of the lipid nanoparticle by the dendritic cells, B cells, and macrophages in the spleen of the mice administered the lipid nanoparticles of Examination Plots B-1 to B-8 in Example 2.
[FIG. 7] FIG. 7 is a diagram showing the results of measuring the Nluc expression level in the dendritic cells, B cells, and macrophages in the spleen of the mice administered the lipid nanoparticles of Examination Plots B-1 to B-8 in Example 2.
[FIG. 8] FIG. 8 is a diagram showing the results of measuring the Nluc expression level in the dendritic cells in the spleen of the mice administered the lipid nanoparticles of Examination Plots A-1 to A-14 and Examination Plots B-1 to B-8 in Example 1 and 2 .
[FIG. 9] FIG. 9 is a diagram showing (A) the relationship between the level of lipid nanoparticle uptake by the splenic dendritic cells and the I-A / I-E expression level, and (B) the relationship between the Nluc gene expression level and the I-A / I-E expression level in the splenic dendritic cells, in the mice administered the lipid nanoparticles of Examination Plots A-1 to A-14 and Examination Plots B-1 to B-8 in Example 1 and 2.
[FIG. 10] FIG. 10 is a diagram showing (A) the relationship between the level of lipid nanoparticle uptake by the splenic dendritic cells and I-A / I-E expression level, and (B) the relationship between the Nluc gene expression level and the I-A / I-E expression level in the dendritic cells, in the mice administered the lipid nanoparticles of Examination Plots A-11, A-15 and A1-16 in Example 3.
[FIG. 11] FIG. 11 is a diagram showing the changes in (A) the ζ average particle diameter (nm), and (B) RNA-encapsulation efficiency (%) over time when the lipid nanoparticles of Examination Plots A-11, A-15 and A1-16 was stored at 4 °C in Example 3.

[FIG. 12] FIG. 12 is a diagram showing the results of measuring over time the tumor volume of the mice who was administered the OVA-mRNA-loaded lipid nanoparticle (mOVA-A11-LNP) in advance and then received a subcutaneous implantation of E.G7-OVA cells in Example 4.

[FIG. 13] FIG. 13 is a diagram showing the results of measuring over time the tumor volume of the mice who received a subcutaneous implantation of E.G7-OVA cells and then administered mOVA-A11-LNP in Example 4.

[FIG. 14] FIG. 14 is a diagram showing the results of measuring over time the tumor volume of the mice who received the second E.G7-OVA cell transplantation in Example 4.

[FIG. 15] FIG. 15 is a diagram showing the results of measuring over time the tumor volume of the mice who received subcutaneous implantation of E.G7-OVA cells and then administered mOVA-A11-LNP in Example 4.

[FIG. 16] FIG. 16 is a diagram showing the results of measuring Nluc expression level in the liver, spleen, lung, kidney and inguinal lymph node of mice administered each of the lipid nanoparticles in Example 5.

[FIG. 17] FIG. 17 is a diagram showing (A) the results of measuring the proportion of EGFP-positive cells (%) to total splenic dendritic cells, (B) the average EGFP fluorescence intensity of the splenic dendritic cells, and (C) the I-A / I-E expression level, in the mice administered either of the lipid nanoparticles in Example 5.

[FIG. 18] FIG. 18 is a diagram showing the results of measuring over time the tumor volume of the mice who received a subcutaneous implantation of E.G7-OVA cells and then administered either of the lipid nanoparticles in Example 6.

DESCRIPTION OF EMBODIMENTS

Hereinbelow, the embodiments of the present invention are to be explained in specific. Herein, "from X1 to X2 (X1 and X2 are real numbers that satisfy X1 < X2)" means "equal to or greater than X1 and equal to or less than X2".

The lipid nanoparticle according to the present invention has a number average particle diameter that is equal to or greater than 150 nm. In immunocytes, macropinocytotic and phagocytotic routes for incorporating relatively large extracellular substances have been developed. The lipid nanoparticle according to the present invention is incorporated into a splenic dendritic cell at a high efficiency because it has a number average particle diameter as large as that is equal to or greater than 150 nm. The number average particle diameter of the lipid nanoparticle according to the present invention is not particularly limited as long as it is equal to or greater than 150 nm, and is preferably from 150 to 700 nm, more preferably from 150 to 600 nm, further preferably from 200 to 600 nm, yet further preferably from 300 to 600 nm, and particularly preferably from 400 to 600 nm.

Here, the average particle diameter of a lipid nanoparticle means a number average particle diameter measured by a dynamic light scattering (DLS) unless otherwise described. A measurement by the dynamic light scattering method can be performed conventionally using a commercially available DLS apparatus, etc.

The Polydispersion Index (PdI) of the lipid nanoparticle according to the present invention is approximately from 0.01 to 0.7, preferably approximately from 0.01 to 0.5, further preferably approximately from 0.01 to 0.2. The zeta potential can be in a range between 1 mV and 20 mV, preferably in a range between 5 mV and 15 mV.

The lipid nanoparticle according to the present invention comprises a pH-sensitive cationic lipid and a polyalkylene glycol-modified lipid. The proportion of the pH-sensitive cationic lipid content to the total amount of the lipids constituting the lipid nanoparticle is from 40 to 70 mol %, and the proportion of the polyalkylene glycol-modified lipid content to the total amount of the lipids constituting the lipid nanoparticle is from 0.5 to 1.75 mol %.

The lipid nanoparticle according to the present invention has both good blood-retentivity and endosome-escaping ability because it comprises a pH-sensitive cationic lipid as a constituent lipid component. In the lipid nanoparticle according to the present invention, the proportion of the pH-sensitive cationic lipid content to the total amount of the lipids constituting the lipid nanoparticle ([the amount of the pH-sensitive cationic lipid (mol)] / ([the amount of the total lipids that constitute the lipid nanoparticle (mol)]) x 100 %) is not particularly limited as long as it is from 40 to 70 mol %, though it is preferably from 40 to 65 mol %, and more preferably from 40 to 60 mol %.

The lipid nanoparticle according to the present invention can comprise only one, or two or more types of pH-sensitive cationic lipid(s) as (a) constituent(s). When there are two or more types of pH-sensitive cationic lipids that constitute the lipid nanoparticle according to the present invention, the amount of the pH-sensitive cationic lipid means the total amount of the lipid molecules corresponding to the pH-sensitive cationic lipid among the lipid molecules constituting the lipid nanoparticle.

As the pH-sensitive cationic lipid, for example, a lipid expressed by the following general formula (I) (hereinbelow may referred to as "pH-sensitive cationic lipid (I) ") is preferably used.
[Chem. 2]

$$(R^1)\ (R^2)C(OH)\text{-}(CH_2)a\text{-}(O\text{-}CO)b\text{-}X \qquad (I)$$

In the general formula (I), **a** denotes an integer from 3 to 5, though it is preferably 4.

b denotes 0 or 1. When **b** is 0, the -O-CO-group is absent, meaning it is a single bond.

In the general formula (I), $R^1$ and $R^2$ are each independently denotes a group expressed by the following general formula (A). In the general formula (A), **q** denotes an integer from 1 to 9; **r** denotes 0 or 1; **s** denotes an integer from 1 to 3; **t** denotes 0 or 1; **u** denotes an integer from 1 to 8; **c** denotes 0 or 1; **v** denotes an integer from 4 to 12; provided that cases are excluded in which both **b** and **c** is 0, **q** is an integer from 3 to 5, **r** and **t** are 1, **s** is 1, and **u + v** is an integer from 6 to 10.

[Chem. 3]

$$CH_3\text{-}(CH_2)q\text{-}(CH{=}CH)r\text{-}(CH_2)s\text{-}(CH{=}CH)t\text{-}(CH_2)u\text{-}(CO\text{-}O)c\text{-}(CH_2)v\text{-} \qquad (A)$$

With respect to the pH-sensitive cationic lipid (I), it is preferred that a hydrocarbon chain of $R^1$ and $R^2$ is a relatively long chain in view of the stability of the lipid nanoparticle. In specific, in the pH-sensitive cationic lipid (I), it is preferred that $R^1$ and $R^2$ are groups having 20 or more carbon atoms. Namely, it is preferred that, in the general formula (A), **q** + 2**r** + **s** + 2**t** + **u** + **c** + **v** is an integer equal to or greater than 19. In particular, with respect to the pH-sensitive cationic lipid (I), **q** + 2**r** + **s** + 2**t** + **u** + **c** + **v** is preferably an integer from 19 to 33, more preferably an integer from 19 to 31, further preferably an integer from 21 to 31, yet further preferably an integer from 21 to 27.

In the pH-sensitive cationic lipid (I), $R^1$ and $R^2$ are, in the context of the general formula (A), preferably groups in which **r** is 1, **t** is 0, **q** is an integer from 5 to 11, preferably an integer from 6 to 10, **s** + **u** is an integer from 5 to 11, preferably an integer from 6 to 10, **c** is 1, **v** is an integer from 4 to 12, and **q** + **s** + **u** + **v** is an integer equal to or greater than 16; groups in which **r** is 0, **t** is 1, **q** + **s** is an integer from 5 to 11, preferably an integer from 6 to 10, **u** is an integer from 5 to 8, **c** is 1, **v** is an integer from 4 to 12, and **q** + **s** + **u** + **v** is an integer equal to or greater than 16; groups in which **r** and **t** are 0, **q** + **s** + **u** is an integer from 13 to 23, preferably from 15 to 21, **c** is 1, **v** is an integer from 4 to 12, and **q** + **s** + **u** + **v** is an integer equal to or greater than 18; groups in which **r** and **t** are 0, **q** + **s** + **u** is an integer from 13 to 23, preferably from 15 to 21, **c** is 1, **v** is an integer from 6 to 10, and **q** + **s** + **u** + **v** is an integer equal to or greater than 18.

In the pH-sensitive cationic lipid (I), $R^1$ and $R^2$ may be any group as long as being represented by the general formula (A). $R^1$ and $R^2$ may be different groups from each other, though preferably they are the same groups.

In the general formula (I), X denotes a group represented by the following general formula (B) or a 5- to 7-membered non-aromatic heterocyclic group. The 5- to 7-membered non-aromatic heterocyclic group denoted by X is bound to (O-CO)b- via a carbon atom.

[Chem. 4]

$$\text{-}(CH_2)d\text{-}N(R^3)(R^4) \qquad (B)$$

In the general formula (B), **d** denotes an integer from 0 to 3. When **d** is 0, $-(CH_2)-$ group is absent, meaning it is a single bond.

In the general formula (B), $R^3$ and $R^4$ are each independently denotes a $C_{1-4}$ alkyl group (an alkyl group having from 1 to 4 carbon atoms) or a $C_{2-4}$ alkenyl group (an alkenyl group having from 1 to 4 carbon atoms). In the $C_{1-4}$ alkyl group or $C_{2-4}$ alkenyl group denoted by $R^3$ and $R^4$, one or two hydrogen atom(s) may have been substituted by (a) phenyl group(s).

The $C_{1-4}$ alkyl group includes a methyl group, ethyl group, propyl group, isopropyl group, n-butyl group, isobutyl group, and t-butyl group. The $C_{2-4}$ alkenyl group includes a vinyl group, 1-propenyl group, 2-propenyl group, 1-methylvinyl group, 2-methyl-1-propenyl group, 1-butenyl group, 2-butenyl group, and 3-butenyl group.

in the general formula (B), $R^3$ and $R^4$ may be bound to each other to form a 5- to 7-membered non-aromatic heterocycle. The 5- to 7-membered non-aromatic heterocycle formed by $R^3$ and $R^4$ bound to each other includes, for example, a 1-pyrrolidinyl group, 1-piperidinyl group, 1-morpholinyl group, and 1-piperazinyl group. In the 5- to 7-membered non-aromatic heterocycle formed by $R^3$ and $R^4$ bound to each other, one or two hydrogen atom(s) in the ring may have been substituted by (a) $C_{1-4}$ alkyl group(s) or (a) $C_{2-4}$ alkenyl group(s). When two hydrogen atoms in the ring have been substituted by (a) $C_{1-4}$ alkyl group(s) or (a) $C_{2-4}$ alkenyl group(s), they may have been substituted by the same groups, or they may be have been substituted by different groups to each other.

In the general formula (I), when X is a 5- to 7-membered non-aromatic heterocyclic group, the heteroatom contained in the heterocyclic group includes a nitrogen atom, oxygen atom or sulfur atom, etc. The heterocycle in the heterocyclic group may comprise as (a) constituent(s) one heteroatom, or two or more heteroatoms, which are the same or different. The heterocycle in the heterocyclic group may be a saturated heterocycle and may comprise one or two or more double bond(s), provided that the heterocycle is never an aromatic ring.

The pKa of the pH-sensitive cationic lipid (I) is not particularly limited, though it can be selected to be, for example, approximately from 4.0 to 9.0, preferably approximately from 4.5 to 8.5, more preferably approximately from 6 to 8, and it is preferred to select the type of each substituent such that it will give a pKa in this range.

The pH-sensitive cationic lipid (I) can easily be produced, for example, by a method specifically shown in Examples herein. A skilled artisan can easily produce any lipid encompassed within the scope of the general formula

(I) by referring to this method for production and appropriately selecting feedstock compounds, reagents and conditions for reactions, etc.

As the pH-sensitive cationic lipids contained in the lipid nanoparticle according to the present invention, above all, a lipid represented by the following general formula (I-1) (hereinbelow mat be referred to as "pH-sensitive cationic lipid (I-1)") is preferred.

[Chem. 5]

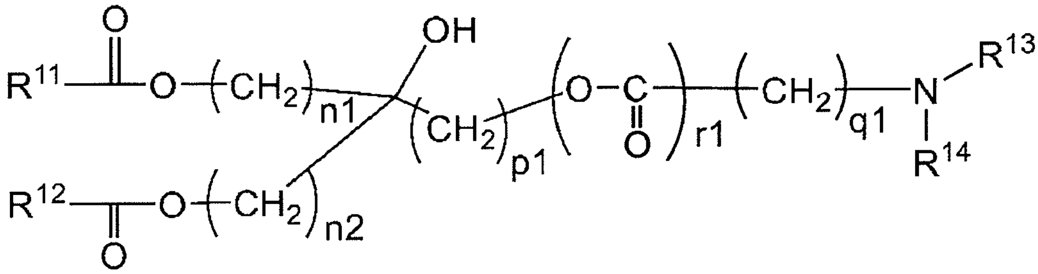

(I-1)

In the general formula (I-1), p1 and q1 denote integers that are equal to or greater than 0 and satisfy p1 + q1 = from 3 to 8, and r1 denotes 0 or 1. When r1 is 0, p1 and q1 are preferably integers that are equal to or greater than 0 and satisfy p1 + q1 = from 3 to 5, particularly preferably integers that are equal to greater than 0 and satisfy p1 + q1 = 4. When r1 is 1, it is preferred that p1 is an integer from 3 to 5 and q1 is an integer from 0 to 2, and it is particularly preferred that p1 is 4 and q1 is an integer from 0 to 2.

In the general formula (I-1), n1 and n2 each independently denotes an integer from 6 to 10. In the pH-sensitive cationic lipid represented by the general formula (I-1), n1 and n2 are preferably 6, 8, or 10, more preferably, 6.

In the general formula (1), $R^{13}$ and $R^{14}$ are each independently a straight-chain $C_{1-3}$ alkyl group or an aryl $C_{1-3}$ alkyl group. The straight-chain $C_{1-3}$ alkyl group includes a methyl group, ethyl group, and n-propyl group. The aryl $C_{1-3}$ alkyl group is preferably a phenyl $C_{1-3}$ alkyl group, particularly preferably a phenylmethyl group (benzyl group).

In the general formula (1), $R^{13}$ and $R^{14}$ may be joined to each other to form a pyrrolidine ring, a piperidine ring, a morpholine ring, or a piperazine ring in which a nitrogen atom may be substituted with a $C_{1-3}$ alkyl group. The nitrogen atom of the piperazine ring formed by $R^{13}$ and $R^{14}$ may have a $C_{1-3}$ alkyl group including a methyl group, ethyl group, *n*-propyl group and isopropyl group, of which a methyl group is preferred. In a preferred compound for the pH-sensitive cationic lipid represented by the general formula (I-1), when r1 is 0, $R^{13}$ and $R^{14}$ are both *n*-propyl groups or they are joined to each other to form a pyrrolidine ring, piperidine ring, morpholine ring or piperazine ring, or a piperazine ring in which a nitrogen atom has been substituted by a methyl group; in a more preferred compound, $R^{13}$ and $R^{14}$ are both n-propyl groups or they are joined to each other to form a morpholine ring.

The pKa of the pH-sensitive cationic lipid represented by the general formula (I-1) is preferably from 4.5 to 7.5, more preferably from 5.0 to 7.1, and further preferably from 5.5 to 6.5. For instance, the pKa of the pH-sensitive cationic lipid in which $R^{13}$ and $R^{14}$ in the general formula (I-1) are both n-propyl groups is approximately 6.25, and the pKa of the pH-sensitive cationic lipid in which $R^{13}$ and $R^{14}$ are joined together to form a morpholine ring is approximately 5.85 (Non-Patent Literature 5).

In the general formula (1), $R^{11}$ and $R^{12}$ are each independently a straight-chain $C_{10-14}$ alkyl group, a straight-chain $C_{10-20}$ alkenyl group having one or two unsaturated bond(s), or $-CH(R^{15})(R^{16})$ ($R^{15}$ and $R^{16}$ are each independently a straight-chain $C_{5-10}$ alkyl group). Among the pH-sensitive cationic lipids represented by the general formula (1), those in which the scaffolding $R^{11}$ and $R^{12}$ are alkyl groups, alkenyl groups or branched-chain alkyl groups with a moderate chain length have a higher scaffold mobility, which improves the transduction efficiency into splenic dendritic cells while suppressing the toxicity to a lower level.

The straight-chain $C_{10-14}$ alkyl group (an alkyl group having from 10 to 14 carbon atoms) includes an *n*-decyl group, *n*-undecyl group, *n*-dodecyl group, *n*-tridecyl group and *n*-tetradecyl group. When $R^{11}$ or $R^{12}$ is a straight-chain $C_{10-14}$ alkyl group, with respect to the pH-sensitive cationic lipid represented by the general formula (I-1), it is preferred that $R^{11}$ and $R^{12}$ are both straight-chain $C_{10-14}$ alkyl groups, and it is more preferred that $R^{11}$ and $R^{12}$ are each independently an *n*-undecyl group, *n*-dodecyl group or *n*-tridecyl group, and it is further preferred that $R^{11}$ and $R^{12}$ are both *n*-undecyl groups, n-dodecyl groups or *n*-tridecyl groups, and it it is yet further preferred that $R^{11}$ and $R^{12}$ are both *n*-tridecyl groups.

The straight-chain $C_{10-20}$ alkenyl group (an alkenyl group having from 10 to 20 carbon atoms) having one or two unsaturated bond(s) may be any group in which one or two of the saturated bond(s) between carbon atoms of the alkyl chain in the straight-chain $C_{10-20}$ alkyl group (the alkyl group having from 10 to 20 carbon atoms) has/have been

changed to (an) unsaturated bond(s). A group in which one or two of the saturated bond(s) between carbon atoms close to the middle of the straight-chain $C_{10-20}$ alkyl group has/have been changed to (an) unsaturated bond(s) is preferred, and a group in which one or two of the saturated bond(s) between carbon atoms close to the middle of the straight-chain $C_{13-18}$ alkyl group (the alkyl group having from 13 to 18 carbon atoms) has/have been changed to (an) unsaturated bond(s) is more preferred. The alkyl group having from 10 to 20 carbon atoms includes an *n*-decyl group, *n*-undecyl group, *n*-dodecyl group, *n*-tridecyl group, *n*-tetradecyl group, *n*-pentadecyl group, *n*-hexadecyl group, *n*-heptadecyl group, *n*-octadecyl group, *n*-nonadecyl group and *n*-eicosyl group. The group in which one or two of the saturated bond(s) between carbon atoms closed the middle of the straight-chain $C_{13-18}$ alkyl group has/have been changed to (an) unsaturated bond(s) is more preferably a group in which one or two saturated bond(s) between carbon atoms close to the middle of a *n*-tridecyl group, *n*-tetradecyl group, *n*-pentadecyl group, *n*-hexadecyl group, *n*-heptadecyl group or *n*-octadecyl group has/have been changed to (an) unsaturated bond(s); which includes a 5-tridecenyl group, 6-tridecenyl group, 7-tridecenyl group, 8-tridecenyl group, 9-tridecenyl group, 5-tetradecenyl group, 6-tetradecenyl group, 7-tetradecenyl group, 8-tetradecenyl group, 9-tetradecenyl group, 6-pentadecenyl group, 7-pentadecenyl group, 8-pentadecenyl group, 9-pentadecenyl group, 10-pentadecyl group, 6-hexadecenyl group, 7-hexadecenyl group, 8-hexadecenyl group, 9-hexadecenyl group, 10-hexadecyl group, 6-heptadecenyl group, 7-heptadecenyl group, 8-heptadecenyl group, 9-heptadecenyl group, 10-heptadecenyl group, 11-heptadecenyl group, 12-heptadecenyl group, 9,12-heptadecenyl group, 7-octadecenyl group, 8-octadecenyl group, 9-octadecenyl group, 10-octadecenyl group, 11-octadecenyl group, 7-nonadecenyl group, 8-nonadecenyl group, 9-nonadecenyl group, 10-nonadecenyl group, 11-nonadecenyl group, 8-eicosenyl group, 9-eicosenyl group, 10-eicosenyl group, 11-eicosenyl group, 12-eicosenyl group, etc.

When $R^{11}$ or $R^{12}$ is a straight-chain $C_{10-20}$ alkenyl group, with respect to the pH-sensitive cationic lipid represented by the general formula (I-1), it is preferred that $R^{11}$ and $R^{12}$ are both straight-chain $C_{10-20}$ alkenyl groups, and it is more preferred that $R^{11}$ and $R^{12}$ are each independently a group in which one or two of the saturated bond(s) between carbon atoms close to the middle of the straight-chain $C_{13-18}$ alkyl group has/have been changed to (an) unsaturated bond(s), and it is further preferred that $R^{11}$ and $R^{12}$ are each independently a 6-hexadecenyl group, 7-hexadecenyl group, 8-hexadecenyl group, 9-hexadecenyl group, 10-hexadecyl group, 6-heptadecenyl group, 7-heptadecenyl group, 8-heptadecenyl group, 9-heptadecenyl group, 10-heptadecenyl group, 11-heptadecenyl group, 12-heptadecenyl group, 9,12-heptadecenyl group, 7-octadecenyl group, 8-octadecenyl group, 9-octadecenyl group, 10-octadecenyl group or 11-octadecenyl group, and it is yet further preferred that $R^{11}$ and $R^{12}$ are each independently a 6-heptadecenyl group, 7-heptadecenyl group, 8-heptadecenyl group, 9-heptadecenyl group, 10-heptadecenyl group, 11-heptadecenyl group, 12-heptadecenyl group or 9,12-heptadecenyl group, and it is particularly preferred that $R^{11}$ and $R^{12}$ are both 8-heptadecenyl groups.

The moiety $-CH(R^{15})(R^{16})$ ($R^{15}$ and $R^{16}$ are each independently a straight-chain $C_{5-10}$ alkyl group) includes, for example, $-CH(-C_5H_{11})(-C_7H_{15})$, $-CH(-C_6H_{13})(-C_8H_{17})$, $-CH(-C_7H_{15})(-C_9H_{19})$ and $-CH(-C_8H_{17})(-C_{10}H_{21})$, etc. When $R^{11}$ or $R^{12}$ is $-CH(R^{15})(R^{16})$, with respect to the pH-sensitive cationic lipid represented by the general formula (I-1), it is preferred that $R^{11}$ and $R^{12}$ are both $-CH(R^{15})(R^{16})$, and it is more preferred that $R^{11}$ and $R^{12}$ are each independently $-CH(-C_5H_{11})(-C_7H_{15})$, $-CH(-C_6H_{13})(-C_8H_{17})$, $-CH(-C_7H_{15})(-C_9H_{19})$, or $-CH(-C_8H_{17})(-C_{10}H_{21})$, and it is further preferred that $R^{11}$ and $R^{12}$ are both $-CH(-C_6H_{13})(-C_8H_{17})$.

In a preferred compound as the pH-sensitive cationic lipid represented by the general formula (I-1), $R^{11}$ and $R^{12}$ are each independently any of those groups in which one or two of the saturated bond(s) between carbon atoms close to the middle of the straight-chain $C_{13-18}$ alkyl group has/have been changed to (an) unsaturated bond(s), or in which $R^{11}$ and $R^{12}$ are each independently *n*-undecyl group, *n*-dodecyl group or *n*-tridecyl group, and p is from 3 to 5. In a more preferred compound, $R^{11}$ and $R^{12}$ are each independently any of those groups in which one or two of the saturated bond(s) between carbon atoms close to the middle of the straight-chain $C_{13-18}$ alkyl group has/have been changed to (an) unsaturated bond(s), and p is from 3 to 5. In a further preferred compound, $R^{11}$ and $R^{12}$ are each independently a 6-heptadecenyl group, 7-heptadecenyl group, 8-heptadecenyl group, 9-heptadecenyl group, 10-heptadecenyl group, 11-heptadecenyl group, 12-heptadecenyl group or 9,12-heptadecenyl group, and p is from 3 to 5. In a yet further preferred compound, $R^{11}$ and $R^{12}$ are both 8-heptadecenyl group and p is from 3 to 5. In a particularly preferred compound, $R^{11}$ and $R^{12}$ are both 8-heptadecenyl group, $R^{13}$ and $R^{14}$ are both methyl group, and p is 4.

Also, in a preferred compound as the pH-sensitive cationic lipid represented by the general formula (I-1), $R^{11}$ and $R^{12}$ are each independently $-CH(R^{15})(R^{16})$, and p is from 3 to 5. In a more preferred compound, $R^{11}$ and $R^{12}$ are each independently $-CH(-C_5H_{11})(-C_7H_{15})$, $-CH(-C_6H_{13})(-C_8H_{17})$, $- CH(-C_7H_{15})(-C_9H_{19})$ or $-CH(-C_8H_{17})(-C_{10}H_{21})$, and p is from 3 to 5. In a further preferred compound, $R^{11}$ and $R^{12}$ are both $-CH(-C_6H_{13})(-C_8H_{17})$, and p is from p is from 3 to 5. In a yet further preferred compound, $R^{11}$ and $R^{12}$ are both $-CH(-C_6H_{13})(-C_8H_{17})$, and p is 4.

The pH-sensitive cationic lipid represented by the general formula (I-1) can easily be produced, for example, by a method specifically shown in Examples herein. A skilled artisan can easily produce any lipid encompassed within the scope of the general formula (I-1) by referring to this method for production and appropriately selecting feedstock compounds, reagents and conditions for reactions, etc.

The lipid nanoparticle according to the present invention has a polyalkylene glycol-modified lipid as a lipid

constituent, by which the surface of the lipid nanoparticle is modified with the polyalkylene glycol. The surface modification with the polyalkylene glycol, which is a hydrophilic polymer, can improve the stability of the lipid nanoparticle such as the retentivity in blood. However, an excessively high density of the polyalkylene glycol on the lipid surface will provide the surface with a high stabilizing ability, resulting in a tendency of the particle diameter of the lipid nanoparticle being small. In the lipid nanoparticle according to the present invention, the proportion of the polyalkylene glycol-modified lipid content to the total amount of the lipids constituting the lipid nanoparticle ([the amount of the polyalkylene glycol-modified lipid (mol)] / ([the amount of the total lipids that constitute the lipid nanoparticle (mol)]) x 100 %) is from 0.5 to 1.75 mol %, so that the stability is high and the number average particle diameter relatively large as 150 nm or greater.

In the lipid nanoparticle according to the present invention, the proportion of the polyalkylene glycol-modified lipid content to the total amount of the lipids constituting the lipid nanoparticle is not particularly limited as long as it is from 0.5 to 1.75 mol %, and it is preferably from 0.5 to 1.6 mol %, and more preferably from 0.5 to 1.5 mol %. The lipid nanoparticle according to the present invention may comprise as (a) constituent(s) only one, or two or more types of polyalkylene glycol-modified lipid(s). When there are two or more types of polyalkylene glycol-modified lipids that constitute the lipid nanoparticle according to the present invention, the amount of the polyalkylene glycol-modified lipid means the total amount of the lipid molecules corresponding to the polyalkylene glycol-modified lipid among the lipid molecules constituting the lipid nanoparticle.

The polyalkylene glycol that can be used includes, for example, a polyethylene glycol, polypropylene glycol, polytetramethylene glycol, polyhexamethylene glycol, etc. The molecular weight of the polyalkylene glycol is, for example, approximately from 300 to 10,000, preferably approximately from 500 to 10,000, further preferably approximately from 1,000 to 5,000.

For instance, in order to modify a lipid with a polyethylene glycol, a stearylated polyethylene glycol (e.g., PEG45 stearate (STR-PEG45), etc.) can be used. In addition, polyethylene glycol derivatives can also be used, such as N-[carbonyl-methoxypolyethylene glycol-2000]-1,2-dipalmitoyl-sn-glycero-3-phosphoethanol amine, n-[carbonyl-methoxypolyethylene glycol-5000]-1,2-dipalmitoyl-sn-glycero-3-phosphoethanol amine, N-[carbonyl-methoxypolyethylene glycol-750]-1,2-distearoyl-sn-glycero-3-phosphoethanol amine, N-[carbonyl-methoxypolyethylene glycol-2000]-1,2-distearoyl-sn-glycero-3-phosphoethanol amine, 1,2-distearoyl-rac-glycero-3-methoxypolyethylene glycol-2000(PEG2k-DSG), 1,2-dimyristoyl-rac-glycero-3-methoxypolyethylene glycol-2000 (PEG2k-DMG), though the polyalkylene glycol-modified lipid is not to be limited thereto.

Among the lipids that constitute the lipid nanoparticle according to the present invention, in addition to the pH-sensitive cationic lipid and the polyalkylene glycol-modified lipid, lipids that are generally used in forming a liposome can be used. Such lipids include, for example, phospholipids, sterols, glycolipids, or saturated or unsaturated fatty acids, etc. These can be used alone or in combination of two or more.

Phospholipids can include glycerophospholipids such as phosphatidyl serine, phosphatidyl inositol, phosphatidyl glycerol, phosphatidyl ethanol amine, phosphatidyl choline, cardiolipin, plasmalogen, ceramide phosphoryl glycerol phosphate, and phosphatidic acid; and sphingophospholipids such as sphingomyelin, ceramide phosphoryl glycerol, and ceramide phosphoryl ethanol amine, etc. Moreover, phospholipids derived from natural ingredients such as egg yolk lecithin and soybean lecithin can also be used. The fatty acid residue in the glycerophospholipid and sphingophospholipid is not particularly limited, though it includes, for example, a saturated or unsaturated fatty acid residue having from 12 to 24 carbon atoms, of which a saturated or unsaturated fatty acid residue having from 14 to 20 carbon atoms is preferred. In specific, it includes an acyl group derived from a fatty acid such as lauric acid, myristic acid, palmitic acid, palmitoleic acid, stearic acid, oleic acid, linoleic acid, linolenic acid, arachidic acid, arachidonic acid, behenic acid and lignoceric acid. When the glycerolipid or sphingolipid has two or more fatty acid residues, the fatty acids residues may all be the same groups, or they may be different groups from each other.

Sterols include, for example, animal-derived sterols such as cholesterol, cholesterol succinic acid, lanosterol, dihydrolanosterol, desmosterol and dihydrocholesterol; plant-derived sterols (phytosterols) such as stigmasterol, sitosterol, campesterol and brassicasterol; and microorganism-derived sterol such as zymosterol and ergosterol, and the like. Glycolipids include, for example, glyceroglycolipids such as sulfoxy ribosylglyceride, diglycosyl diglyceride, digalactosyl diglyceride, galactosyl diglyceride, and glycosyl diglyceride; sphingoglycolipids such as galactosyl cerebroside, lactosyl cerebroside, and ganglioside. Saturated or unsaturated fatty acids include, for example, saturated or unsaturated fatty acids having from 12 to 20 carbon atoms such as palmitic acid, oleic acid, stearic acid, arachidonic acid and myristic acid.

The lipid nanoparticle according to the present invention preferably comprises a neutral lipid as a constituent lipid, in addition to the pH-sensitive cationic lipid and the polyalkylene glycol-modified lipid. It more preferably comprises one or more selected from a group consisting of phospholipids and sterols, and further preferably comprises a phospholipid and a cholesterol. When the lipid nanoparticle according to the present invention comprises both the phospholipid and the sterol as constituent lipids, the proportion of the phospholipid content to the total amount of the lipids constituting the lipid nanoparticle ([the amount of the phospholipid (mol)] / ([the amount of the total lipids that constitute the lipid nanoparticle (mol)]) x 100 %) is preferably from 5 to 40 mol %, more preferably from 5 to 30 mol %, further preferably

from 5 to 20 mol %, and yet further preferably from 5 to 15 mol %. When the lipid nanoparticle according to the present invention comprises both the phospholipid and the sterol as constituent lipids, the proportion of the sterol content to the total amount of the lipids constituting the lipid nanoparticle ([the amount of the sterol (mol)] / ([the amount of the total lipids that constitute the lipid nanoparticle (mol)]) x 100 %) is preferably from 5 to 40 mol %, more preferably from 10 to 40 mol %, further preferably from 15 to 40 mol %, and yet further preferably from 20 to 40 mol %.

The lipid nanoparticle according to the present invention can undergo an appropriate surface modification, etc., as necessary.

By modifying the surface of the lipid nanoparticle according to the present invention with a hydrophilic polymer, etc., its retentivity in blood can be increased. The surface modification may also be performed by using a lipid that has been modified with these modifying groups as a lipid constituent for the lipid nanoparticle.

Upon producing the lipid nanoparticle according to the present invention, for example, a glycophorin, ganglioside GM1, phosphatidyl inositol, ganglioside GM3, glucuronic acid derivative, glutamic acid derivative, and polyglycerin phospholipid derivative can be utilized as a lipid derivative for increasing the retentivity in blood. Moreover, dextran, pullulan, Ficoll, polyvinyl alcohols, styrene-maleic anhydride alternating copolymers, divinyl ether-maleic anhydride alternating copolymers, amylose, amylopectin, chitosan, mannan, cyclodextrin, pectin, carrageenan, etc., can also be used for the surface modification, as a hydrophilic polymer for increasing the retentivity in blood, in addition to the polyalkylene glycol.

Moreover, in order to promote the nuclear translocation of the lipid nanoparticle according to the present invention, for example, the surface of the lipid nanoparticle can be modified with a trisaccharide or a higher oligosaccharide compound. The type of the trisaccharide or a higher oligosaccharide compound is not particularly limited, however, for example, an oligosaccharide compound in which approximately from 3 to 10 sugar units are bound can be used, and an oligosaccharide compound in which approximately from 3 to 6 sugar units are bound can preferably be used. In particular, an oligosaccharide compound which is from a trimer to a hexamer of glucoses can preferably used, and an oligosaccharide compound which is a trimer or tetramer of glucoses can further preferably be used. More specifically, isomaltotriose, isopanose, maltotriose, maltotetraose, maltopentaose or maltohexaose, etc., can suitably be used, of which further preferred are maltotriose, maltotetraose, maltopentaose or maltohexaose to which glucoses are bound via $\alpha$1-4 linkage. Particularly preferred is maltotriose or maltotetraose, and the most preferred is maltotriose. The level of the surface modification of the lipid nanoparticle with the oligosaccharide compound is not particularly limited, though it is, for example, approximately from 1 to 30 mol %, preferably approximately from 2 to 20 mol %, and more preferably approximately from 5 to 10 mol % in relation to the total lipid amount.

A method for modifying the surface of the lipid nanoparticle with an oligosaccharide compound is not particularly limited. For instance, a liposome lipid nanoparticle whose surface has been modified with a monosaccharide such as galactose or mannose has been known (WO 2007/102481), and the method of surface modification described in this publication can be employed. The entire disclosure of the publication described above is incorporated herein by reference as the disclosure of the specification of the present application.

Moreover, the lipid nanoparticle according to the present invention can be conferred any one or more function(s) such as, for example, a temperature change-sensing function, a membrane-penetrating function, a gene-expressing function and a pH-sensing function. By conferring these functions as appropriate, it is possible to improve the retentivity of the lipid nanoparticle in blood, which enables an efficient endosomal escape by the lipid nanoparticle after endocytosis in the target cell and more efficient expression of the encapsulated nucleic acid in the splenic dendritic cell.

The lipid nanoparticle according to the present invention may comprise one or more substance(s) selected from a group consisting of antioxidants such as tocopherol, propyl gallate, ascorbyl palmitate or butylated hydroxytoluene, charged substances, and membrane polypeptides, etc. A charged substance that gives a positive charge can include, for example, saturated or unsaturated aliphatic amines such as stearylamine and oleylamine. A charged substance that gives a negative charge include, for example, dicetyl phosphate, cholesteryl hemisuccinate, phosphatidyl serine, phosphatidyl inositol and phosphatidic acid. Membrane polypeptides include, for example, membrane superficial polypeptides or membrane intrinsic polypeptides. The amounts of these substances combined are not particularly limited, and they are appropriately selected for the purpose.

The form of the lipid nanoparticle according to the present invention is not particularly limited, though it can includes, for example, a single-membrane liposome, multilayered liposome or spherical micelle in a form being dispersed in an aqueous solvent, or an amorphous layered structure, etc.

The lipid nanoparticle according to the present invention preferably includes an ingredient of interest to be delivered into the target cell within the particle which is covered by a lipid membrane. The ingredient that the lipid nanoparticle according to the present invention includes within the particle is not particularly limited as long as it has a size that can be included. The lipid nanoparticle according to the present invention can encapsulate any substance such as a nucleic acid, a saccharide, a peptide, a low molecular weight compound and a metal compound.

A preferred ingredient to be included in the lipid nanoparticle according to the present invention is a nucleic acid. The nucleic acid may be a DNA or an RNA, including analogues or derivatives thereof (e.g., a peptide nucleic acid (PNA) and phosphorothioated DNA, etc.). The nucleic acid to be included in the lipid nanoparticle according to the

present invention may be a single-stranded nucleic acid or double-stranded nucleic acid, and may be in a linear or cyclic form. The nucleic acid to be included in the lipid nanoparticle according to the present invention is preferably an mRNA or plasmid DNA (pDNA) particularly for their good introduction efficiency into splenic dendritic cells.

The nucleic acid to be included in the lipid nanoparticle according to the present invention preferably comprises an exogenous gene to be expressed in the target cell. More preferably, it is a nucleic acid that is incorporated into a cell and thereby functions to express the exogenous gene in the cell. The exogenous gene may be a gene that is intrinsically included in the genome DNA of the target cell (preferably a splenic dendritic cell), or a gene that is not included in the genome DNA. Such nucleic acid includes a gene expression vector comprising a nucleic acid that consists of a nucleotide sequence encoding the gene of interest to be expressed. The gene expression vector may be one that is present as an extrachromosomal gene in the cell into which it is introduced, or may be one that is incorporated into the genome DNA by homologous recombination.

A gene expression vector to be included in the lipid nanoparticle according to the present invention is not particularly limited, and vectors that are generally used in gene therapy, etc. can be used. The gene expression vector to be included in the lipid nanoparticle according to the present invention is preferably a nucleic acid vector such as a plasmid vector. The plasmid vector may be in a cyclic as it is, or it may be cut beforehand into a linear form before being encapsulated into the lipid nanoparticle according to the present invention. The gene expression vector can be designed by a conventional method utilizing molecular biological tools that are generally used, based on the nucleotide sequence information of the target gene to be expressed, and can be produced by various known methods.

It is also preferred that the nucleic acid to be included in the lipid nanoparticle according to the present invention is an mRNA encoding a peptide or protein to be translated and expressed in the target cell. The peptide, etc. encoded by the mRNA may be a protein or a partial protein thereof that is encoded by the gene that is intrinsically included in the genome DNA of the target cell (preferably a splenic dendritic cell), or may be a protein or a partial protein thereof whose gene is not included in the genome DNA of the target cell. The mRNA is not particularly limited in its structure as long as it can be translated in the target cell into which it is introduced, though it preferably has similar structures to the native mRNA. The structures include, for example, a 5' cap structure, 3' poly(A) structure, 5' untranslated region, 3' untranslated region, etc. The mRNA to be included in the lipid nanoparticle according to the present invention can be designed by a conventional method utilizing molecular biological tools that are generally used, based on the amino acid sequence information for the peptide, etc., of interest to be expressed in the target cell or the nucleotide sequence information encoding the peptide, etc., and can be produced by various known methods.

It is also preferred that the nucleic acid to be included in the lipid nanoparticle according to the present invention is a functional nucleic acid to control the expression of the target gene that is present in the target cell. Such a functional nucleic acid includes an antisense oligonucleotide, an antisense DNA, an antisense RNA, an siRNA, a microRNA, an mRNA, etc. It may also be a pDNA that can be an siRNA expression vector to express an siRNA in a cell. The siRNA expression vector can be prepared from a commercially available siRNA expression vector, which may also be altered as appropriate.

The method for producing a lipid nanoparticle according to the present invention is not particularly limited, and any method that can be available to a skilled artisan can be employed. As an example, it can be produced by dissolving all lipid components in an organic solvent such as chloroform; forming a lipid membrane by exsiccating the mixture under reduced pressure using an evaporator or by spray-drying it using a spray drier; and subsequently adding an aqueous solvent comprising the ingredient to be encapsulated in the lipid nanoparticle (e.g., a nucleic acid, etc.) to the mixture dried as described above; and further emulsifying it using a emulsifier such as a homogenizer, a ultrasonic emulsifier or a high-pressure splaying emulsifier, etc. It may also be produced by a method that is well known as a method for producing a liposome, for example, the reverse-phase evaporation method, etc. When it is desired to control the size of the liposome, an extrusion (extruding filtration) may be performed under a high pressure using a membrane filter having uniform pore diameter, etc.

The composition of the aqueous solvent (dispersion medium) is not particularly limited, and can include, for example, buffers such as a phosphate buffer, citrate buffer and phosphate buffered physiological saline, a physiological saline, and a medium for cell-culturing, etc. These aqueous solvents (dispersion media) can stably disperse the lipid nanoparticles, though, furthermore, sugars (solutions) such as monosaccharides (e.g., glucose, galactose, mannose, fructose, inositol, ribose and xylose), disaccharides (e.g., lactose, sucrose, cellobiose, trehalose and maltose), trisaccharides (e.g., raffinose and melezitose), polysaccharides (e.g., cyclodextrin), and a sugar alcohol (e.g., erythritol, xylitol, sorbitol, mannitol and maltitol), and a polyhydric alcohol (solution) such as glycerin, diglycerin, polyglycerin, propylene glycol, polypropylene glycol, ethylene glycol, diethylene glycol, triethylene glycol, polyethylene glycol, ethylene glycol monoalkyl ether, diethylene glycol monoalkyl ether and 1,3-butylene glycol, etc., may be added. In order to stably preserve the lipid nanoparticle dispersed in this aqueous solvent for a prolonged period, it is desirable to exclude the electrolytes in the aqueous solvent to the utmost, from a viewpoint of physical stability (e.g., for suppressing an aggregation). Moreover, from a viewpoint of chemical stability of the lipid, it is desirable to set the pH of the aqueous solvent from weak acid to near neutral (approximately from pH 3.0 to 8.0), and/or to eliminate dissolved oxygen by bubbling

with nitrogen gas, etc.

The lipid nanoparticle according to the present invention can be produced by the alcohol dilution method using a flow path. The flow path to be used in the production may be a three-dimensional micromixer-installed micro flow path that is capable of achieving instant mixture of two liquids. However, it is preferable to use a flow path structure having a simple two-dimensional structure provided with a micro-sized flow path through which feedstock solutions are to be run and in which baffles (baffle plate) of specified widths in relation to the width of the flow path are alternately disposed from the two side faces, as described in Patent Literature 2, because it can form a system for forming nano-sized lipid particles with high particle-diameter controllability.

In specific, a flow path structure as shown in FIG. 1 (hereinbelow, may be referred to as "the flow path structure of the present invention") is preferably used. In its upstream side (the left side of the figure), a first introduction path 10 for introducing a first fluid and a second introduction path 20 for introducing a second fluid, which are independent to each other and each has a specified length, join together to form one dilution flow path 30 towards their downstream. The the dilution flow path 30 has a flow path part 50, which is two-dimensionally bent at least in a part thereof. When the axis direction of the dilution flow path upstream to the flow path part 50 toward the direction of the extension of the axis is designated to be the X direction; the width direction of the dilution flow path that perpendicularly intersects this X direction is designated to be the Y direction; and the width of the dilution flow path upstream to the the flow path part 50 is designated to be $y_0$, the bent flow path part 50 is formed by providing at least two or more structural elements 40 with specified intervals $d_1$, $d_2$, ..., wherein the two or more structural elements protrude alternately from the both side faces of the dilution flow path opposing in the Y direction toward the center of the flow path, approximately in the Y direction (approximately in + Y direction and approximately in - Y direction), wherein the two or more structural elements 40 have specified heights $h_1$, $h_2$, ... that are equal to or greater than 1/2 $y_0$ and smaller than 1 $y_0$, and have specified widths $x_1$, $x_2$, ..., in the X direction, thereby defining the width of the dilution flow path. Namely, a part where said structural element 40 is present, the flow path widths $y_1$, $y_2$, ... of the dilution flow path are defined to be equal to or less than 1/2 $y_0$, or equal to or less than 1/2 $y_0$ and equal to or more than 1/40 $y_0$, in particular, for specified lengths $x_1$, $x_2$, ..., in the X direction.

The flow path structure of the present invention is conceptionally in a shape in which approximately rectangle-shaped baffles are alternately disposed from the both side faces in a micro-sized flow path, as illustrated in FIG. 1 and explained above. However, it is noted that the flow path structure of the present invention is actually not limited to one that is constructed by disposing baffles of different bodies on the flow path in this way. Namely, the structure of the structural element 40 is not particularly limited as long as a flow path is formed that has a similar shape corresponding to the flow path formed by disposing such baffles. The walls of the flow path structure can be integrally formed while bending them into a specified shape (and keeping a specified thickness at the same time) so as to construct the structural element 40 as described above, constructing a flow path shape of a two-dimensional structure that is capable of bending and expanding as defined above. Such an embodiment is naturally encompassed in the flow path structure of the present invention. A flow path of such a two-dimensional structure can relatively easily be formed, for example, by injection molding, cast molding, or shaping using a 3D printer, using a thermoplastic resin, a thermosetting resin, an ultraviolet curing resin, a metallic or glassy material, etc.

The flow path width $y_0$ of the dilution flow path 30 after the first introduction path 10 and the second introduction path 20 join together will depend to some extent on the size of the particle diameter of the nano-sized lipid particles to be formed, though, typically, it is preferably approximately from 20 to 1000 $\mu$m, more preferably approximately from 100 to 200 $\mu$m. In order to obtain a desired nano-sized lipid particles, specifically, for example, those of a particle diameter in a range approximately from 10 to 100 nm, it is a required condition to some extent that a lipid solution is diluted with a dilution medium in a the flow path having a width $y_0$ in the above-described range.

Each of the heights $h_1$, $h_2$, ..., of the structural elements 40 (the length in the Y direction) is, in relation to the flow path width $y_0$ of the dilution flow path 30 on its upstream side, equal to or greater than 1/2 $y_0$ and less than 1 $y_0$, preferably equal to or greater than 1/2 $y_0$ and equal to or less than 39/40 $y_0$, further preferably equal to or greater than 112 $y_0$ and equal to or less than 3/4 $y_0$. By the presence of each structural element 40, in relation to the flow path width $y_0$ of the dilution flow path 30 on its upstream side, the flow path widths $y_1$, $y_2$, ..., are decreased to a width of less than 1/2 $y_0$ and greater than 0. It is noted that the heights $h_1$, $h_2$, ..., of the multiple structural elements 40 provided in the bent flow path part 50 are not required to be identical, and may each be different as long as it satisfies specified conditions as mentioned above. The flow path widths $y_1$, $y_2$, ..., formed in this way may also be different from each other. For instance, in an embodiment, the widths $h_1$, $h_2$, ..., of the structural elements 40 may gradually be increased as they go downstream, narrowing the flow path widths $y_1$, $y_2$, ... By providing defined heights $h_1$, $h_2$, ..., of the structural elements 40 (the lengths in the Y direction) and limiting the flow path widths $y_1$, $y_2$, ..., of the parts where the structural elements 40 are present to less than 1/2 $y_0$, the molecular diffusion efficiency will be improved.

Each of the heights $h_1$, $h_2$, ..., of the structural elements 40 will also depend on other conditions such as the size of the lipid particle to be obtained, the number of the structural elements 40, each of the widths (the lengths in X direction) $x_1$, $x_2$, ..., of the mixer structural elements 40, and the intervals $d_1$, $d_2$, ..., between adjacent structural elements

40, and is not particularly limited. Nevertheless, specifically, it is desirable that, for example, when the flow path width $y_0$ of the upstream dilution flow path is 200 μm, each of the heights $h_1$, $h_2$, ..., of the structural elements 40 is 100 μm or greater and less than 200 μm. Therefore, each of the flow path widths $y_1$, $y_2$, ..., at positions where the structural elements 40 are present is approximately less than 100 μm, which is a width that is less than 1/2 $y_0$ and greater than 0.

Moreover, each of the widths $x_1$, $x_2$, ..., of the structural elements 40 (the lengths in the X direction) will depend on other conditions such as the size of the lipid particle to be obtained, the number of the structural elements 40, each of the heights $h_1$, $h_2$, ..., of the structural elements 40 (the lengths in the Y direction), and the intervals $d_1$, $d_2$, ..., between adjacent structural elements 40. Nevertheless, it is preferred that each of the widths $x_1$, $x_2$, ..., of the structural elements 40 (the lengths in the X direction) is approximately equal to or greater than 1/10 $y_0$ and equal to or less than 5 $y_0$ in length in relation to the flow path width $y_0$ of the upstream dilution flow path. In specific, for example, when the flow path width $y_0$ of the upstream dilution flow path is from 20 to 1000 μm, typically 200 μm, it is desirable that each of the widths $x_1$, $x_2$, ..., of the structural elements 40 is approximately from 20 μm to 1000 μm. The widths $x_1$, $x_2$, ..., of the structural elements 40 are not required to be identical, and may each be different as long as it satisfies specified conditions as described above. For instance, in an embodiment, the widths $x_1$, $x_2$, ..., may gradually be increased as they go downstream.

Moreover, each of the intervals $d_1$, $d_2$, ..., between adjacent structural elements 40 will depend on other conditions such as the size of the lipid particle to be obtained, the number of the structural elements 40, each of the heights $h_1$, $h_2$, ..., of the structural elements 40 (the lengths in the Y direction), and each of the widths $x_1$, $x_2$, ..., of the structural elements 40 (the length in the X direction). Nevertheless, it is preferred that each of the intervals $d_1$, $d_2$, ..., between adjacent structural elements 40 is approximately equal to or greater than 1/10 $y_0$ and equal to or less than 5 $y_0$ in relation to the flow path width $y_0$ of the upstream dilution flow path. In specific, for example, when the flow path width $y_0$ of the upstream dilution flow path is from 20 to 1000 μm, typically 200 μm, it is desirable that each of the intervals $d_1$, $d_2$, ..., between adjacent structural elements 40 is approximately from 20 μm to 1000 μm. The intervals $d_1$, $d_2$, ..., between adjacent structural elements 40 are not required to be identical, and may each be different as long as it satisfies specified conditions as described above. For instance, in an embodiment, the intervals $d_1$, $d_2$, ..., may be decreased as they go downstream.

in the flow path structure of the present invention, when the axis direction of the upstream dilution flow path toward its extension direction is designated to be the X direction, and the width direction of the dilution flow path that perpendicularly intersects this X direction is designated to be the Y direction, each structural element 40 extends alternately from both side faces toward the center of the flow path approximately in the Y direction (approximately in + Y direction and approximately in - Y direction) as described above, and has a wall face that intersects at approximately a right angle to the flow path direction (the X direction). This angle is not required to strictly be 90°, and can be effective as a configuration even if it inclines to some extent. The angle is not particularly limited, though, in specific, it is accepted that the angle is, for example, in a range between 30° and 150°, more desirably between 40° and 140°, particularly desirably between 80° and 100°. Furthermore, it is accepted that the shape of the corner part of each structural element 40 on the side of the flow path center is rounded to some extent. It is not particularly limited, though the roundness of, for example, equal to or less than R50 μm, more desirably equal to or less than R20 μm might be accepted. However, in order to obtain nano-sized lipid particles with higher controllability and uniformity, it is desirable that such permissible differences are as small as possible. Moreover, in an embodiment shown in FIG. 1, the X direction, which is the axis direction of the upstream dilution flow path in the flow path structure toward its extension direction, is expressed for convenience as a straight line, though this X direction merely indicates the axis direction of the dilution flow path, and, in effect, it is not limited to such a straight line and may be curved with certain curvature. In such a case, the Y direction, which is the width direction of the dilution flow path that perpendicularly intersects this X direction will indicate a direction that is perpendicular to the X direction at a position of that unit length.

The flow path structure of the present invention is a two-dimensional flow path structure as described above, and therefore the size of its flow path in depth direction (the direction of paper thickness in FIG. 1) is not particularly limited. Nevertheless, it is preferably approximately from 10 to 1000 μm, more preferably approximately from 50 to 200 μm.

The flow path to be used when a lipid nanoparticle according to the present invention is produced by alcohol dilution method is not particularly limited as long as it is a flow path in which the dilution flow path 30 in the flow path structure shown in FIG. 1 is capable of generating a three-dimensional flow. For instance, the flow path structure of the present invention may be a chaotic micromixer (a staggered herringbone mixer) (Non-Patent Literature 6) which generates a chaotic flow via grooves or microspikes formed on the wall faces of the flow path instead of the two-dimensionally bent flow path part 50, at least in a part the dilution flow path 30.

The dilution in the flow path structure of the present invention depends on molecular dispersion. The faster the speed at which the feedstock lipid solution is diluted, the smaller the particle size of the produced lipid particle. Therefore, it is possible to control the rate at which the feedstock solution is diluted by adjusting the width, length or arrangement of the structural elements (baffles), and it is thereby possible to form a lipid nanoparticle with a higher particle-diameter controllability than the conventional one.

In the flow path structure of the present invention, when a first introduction path 10 for introducing a first fluid and a second introduction path 20 for introducing a second fluid, each of which has a specified length, join together to form one dilution flow path, as shown in FIG. 1, a lipid solution in which a lipid component has been dissolved in ethanol is introduced from the first introduction path 10, and an aqueous solution is introduced from the second introduction path 20, respectively. In the aqueous solution to be introduced from the second introduction path 20, a water-soluble ingredient to be loaded into the lipid nanoparticle is dissolved in advance. For instance, by introducing an aqueous solution containing a nucleic acid (a nucleic acid-containing aqueous solution) from the second introduction path 20, the lipid solution is diluted by the nucleic acid-containing aqueous solution in the dilution flow path, during which process a lipid nanoparticle in which the nucleic acid is loaded is formed (the forming step).

The nucleic acid-containing aqueous solution can be prepared by dissolving a nucleic acid of interest to be loaded in the lipid nanoparticle in an aqueous solvent. The aqueous solvent is not particularly limited as long as it is an aqueous solvent in which the nucleic acid can stably be dissolved and in which the produced lipid nanoparticles can stably be dispersed. The aqueous solvent can include, for example, buffers such as an acetate buffer, citrate buffer, phosphate buffer and phosphate-buffered physiological saline, a physiological saline and a medium for cell-culturing, etc. To these aqueous solvents (dispersion media), monosaccharides (e.g., glucose, galactose, mannose, fructose, inositol, ribose and xylose sugar); disaccharides (e.g., lactose, sucrose, cellobiose, trehalose and maltose); trisaccharides (e.g., raffinose and melezitose); polysaccharides (e.g., cyclodextrin), and a sugar alcohol (e.g., erythritol, xylitol, sorbitol, mannitol and maltitol); polyhydric alcohols such as glycerin, diglycerin, polyglycerin, propylene glycol, ethylene glycol, diethylene glycol, triethylene glycol, polyethylene glycol, ethylene glycol monoalkyl ether, diethylene glycol monoalkyl ether and 1,3-butylene glycol; etc., may further be added.

When the pH of the nucleic acid-containing aqueous solution is acidic, the lipid nanoparticle is formed under an acidic condition. Therefore, the pH-sensitive cationic lipid contained in the lipid nanoparticle will be positively charged, which induces an electrostatic interaction with the negatively charged nucleic acid, resulting in an efficient loading of the nucleic acid into the lipid nanoparticle. Accordingly, for forming a lipid nanoparticle in which a nucleic acid is loaded, it is preferred to dissolve the nucleic acid in an acidic buffer to prepare an aqueous solution containing the nucleic acid. The pH of the acidic buffer is preferably in a range between 3.5 and 6.0, more preferably in a range between 3.8 and 5.5, further preferably in a range between 3.8 and 5.0, and yet further preferably in a range between 3.8 and 4.5. The acidic buffer that can be used is, for example, an acetate buffer, a citrate buffer, etc.

When the pH of the nucleic acid-containing aqueous solution is acidic, it is preferred to provide a dialysis step in which the lipid nanoparticles formed in the flow path structure is dialyzed through a buffer having a neutral pH (pH 6.8 to 7.6) which can be administered into an organism, such as PBS. This step will give a lipid nanoparticle that can relatively safely be administered to an animal.

It is preferred to adjust the nucleic acid-containing aqueous solution such that the sodium chloride (NaCl) concentration is equal to or greater than 280 mM. In general, when using a flow path structure as shown in FIG. 1, the particle diameter of the lipid nanoparticle to be formed will be equal to or less than 100 nm; the majority thereof will be approximately 20 nm. However, when the nucleic acid-containing aqueous solution contains a high concentration of salts, the repulsion between the charges of the nucleic acids will be eased and hydration will be inhibited. As a result, the interface will become instable, which increases the diameter of the formed lipid nanoparticles. Then, by dialyzing the lipid nanoparticles with increased particle diameter through a buffer indicating a neutral pH, lipid nanoparticles will be fused to each other to give a large lipid nanoparticle with a number average particle diameter equal to or greater than 300 nm. The NaCl concentration in the nucleic acid-containing aqueous solution is not particularly limited as long as it is equal to or greater than 280 mM, and it is preferably from 280 to 500 mM, more preferably from 280 to 450 mM, and further preferably from 280 to 420 mM.

The flow path structure of the present invention comprises a plurality of introduction paths that are each independent. These introduction paths may each have a specified length and may join together one dilution flow path. There may be three introduction paths. When the flow path structure of the present invention comprises three introduction paths, the first introduction path, the second introduction path and the third introduction path can each have a specified length and may join together to form one dilution flow path, such that a first fluid introduced from the first introduction path will be in contact with a third fluid introduced from the third introduction path, before the first fluid introduced from the first introduction path joins with a second fluid introduced from the second introduction path. For producing a lipid nanoparticle in which a nucleic acid is loaded, a lipid solution in which a lipid component is dissolved in ethanol is introduced from the first introduction path, the nucleic acid-containing aqueous solution is introduced from the second introduction path, and the aqueous solvent used for the preparation of the nucleic acid-containing aqueous solution is introduced from the third introduction path, respectively.

The aqueous dispersion of the lipid nanoparticles which has been dialyzed as necessary can be preserved in a dried state until its use. When the aqueous dispersion of the lipid nanoparticles is freeze-dried or spray-dried, its stability might be improved, for example, by using sugars (aqueous solutions) such as monosaccharides (e.g., glucose, galactose, mannose, fructose, inositol, ribose and xylose sugar), disaccharides (e.g., lactose, sucrose, cellobiose, trehalose and maltose), trisaccharides (e.g., raffinose and melezitose), polysaccharides (e.g., cyclodextrin), and a sugar alcohol (e.g., erythritol, xylitol, sorbitol, mannitol and maltitol). Moreover, when the above-described dispersion is to be frozen, its stability might be improved, for example, by using the above-described sugars as well as polyhydric alcohols (aqueous solutions) such as glycerin, diglycerin, polyglycerin, propylene glycol, polypropylene glycol, ethylene glycol, diethylene glycol, triethylene glycol, polyethylene glycol, ethylene glycol monoalkyl ether, diethylene glycol monoalkyl ether and 1,3-butylene glycol.

By assembling various reagents, etc., which are used in the production of the lipid nanoparticle according to the present invention into a kit, the lipid nanoparticle according to the present invention can more conveniently be produced. For instance, a preferred kit for producing a nucleic acid-loaded lipid nanoparticle using a flow path structure is provided with a dry matter of a lipid composition comprising all lipid components for constituting the lipid nanoparticle, and a dry matter comprising NaCl to be dissolved in water to prepare an aqueous solution at NaCl concentration equal to or greater than 280 mM. By dissolving the dry matter of a lipid composition comprising all lipid components for constituting the lipid nanoparticle in ethanol, a lipid solution to be introduced into the first flow path of the flow path structure shown in FIG. 1 can conveniently be prepared. Moreover, by mixing the dry matter to be dissolved in water to prepare an aqueous solution at NaCl concentration equal to or greater than 280 mM, water, and the nucleic acid to be loaded into the lipid nanoparticle, a nucleic acid-containing aqueous solution to be introduced into the second flow path of the flow path structure shown in FIG. 1 can conveniently be prepared. The kit preferably further comprises one or more selected from a group consisting of ethanol, a phosphate buffered physiological saline, and a nucleic acid.

When a lipid nanoparticle according to the present invention in which a gene expression vector has been encapsulated is administered to an animal individual, the gene expression vector encapsulated in the lipid nanoparticle will be expressed in a splenic dendritic cell with a high efficiency. Similarly, a lipid nanoparticle according to the present invention in which an mRNA has been encapsulated is administered to an animal individual, the mRNA encapsulated in the lipid nanoparticle will be introduced into a splenic dendritic cell and translated, and a peptide, etc., encoded by the mRNA will be expressed with a high efficiency. Moreover, when a lipid nanoparticle according to the present invention in which an short hairpin RNA (shRNA) expression vector has been encapsulated is administered to an animal individual, the shRNA expression vector encapsulated in the lipid nanoparticle will be expressed in a splenic dendritic cell with a high efficiency and thereby suppress the expression of a gene being targeted by the expression vector.

Due to this gene expression activity that is highly selective to splenic dendritic cells, the lipid nanoparticle according to the present invention functions as a gene expression carrier for targeting a splenic dendritic cell. By encapsulating an exogenous gene or mRNA intended to be expressed in a splenic dendritic cell into the lipid nanoparticle according to the present invention and administering it to a subject animal, the exogenous gene and mRNA is expressed in the splenic dendritic cell of the subject animal. Accordingly, the lipid nanoparticle according to the present invention is useful as an active ingredient for a pharmaceutical composition used in gene therapy. For instance, it is useful as an active ingredient for a pharmaceutical composition used in gene therapy for targeting splenic dendritic cells.

Moreover, the lipid nanoparticle according to the present invention is particularly useful as a nucleic acid vaccine in which a nucleic acid encoding for an antigen has been loaded. The nucleic acid vaccine may be a DNA vaccine, though it is preferably an mRNA vaccine. Because an mRNA vaccine only needs to be introduced into the cytoplasm, it has a higher versatility than a DNA vaccine which requires to be introduced into the cell nucleus. When the lipid nanoparticle according to the present invention is an mRNA vaccine, upon administering the lipid nanoparticle to an animal, the mRNA loaded in the lipid nanoparticle is translated to express an antigen protein in a splenic dendritic cell of the animal. The immune system will be activated by this antigen protein thus expressed. In addition, because an mRNA itself has a strong immunogenicity, it can be expected that an immunity against the antigen protein can be acquired by an mRNA vaccine without concomitantly using an immunoactivator such as an adjuvant. Namely, the lipid nanoparticle according to the present invention is useful as a vaccine that is used in cancer vaccine therapy or in infection therapy.

An animal to which the lipid nanoparticle according to the present invention is administered is not particularly limited, and it may be a human, or may be an animal other than human. Non-human animals include mammals such as cows, pigs, horses, sheep, goats, monkeys, dogs, cats, rabbits, mice, rats, hamsters and guinea pigs, and birds such as chickens, quails and ducks. Moreover, the route of administration for administrating the lipid nanoparticle according to the present invention to an animal is not particularly limited, though it is preferably a parenteral administration such as an intravenous administration, enteral administration, intramuscular administration, subcutaneous administration, transdermal administration, nasal administration and pulmonary administration.

EXAMPLES

Next, the present invention will be described in more detail with reference to Examples, though the present invention is not to be limited by the following Examples.

<Production of RNA-loaded lipid nanoparticles>

In the experiments hereinafter, the nucleic acid-loaded lipid nanoparticles were produced using a lipid nanoparticle producing device ("iLiNP", Lilac pharma Co., Ltd.) comprising a flow path structure as shown in FIG. 1.

An aqueous solution containing a nucleic acid was prepared by dissolving in an acetate buffer solution (25 mM, pH 4.0), in which NaCl has been dissolved as necessary, the nucleic acid to be loaded in the lipid nanoparticle such that the RNA is at 0.142 mg/mL. As the nucleic acid to be loaded in the lipid nanoparticle, a mixture of an mRNA encoding for nano-luciferase (NLuc) (NLuc-mRNA) with an siRNA that targets human polo-like kinase 1 (hPLK1) (a double-stranded RNA: hPLK1-siRNA) that has no murine homologous sequence at NLuc-mRNA: hPLK1-siRNA = 1:19 (weight ratio), unless otherwise described. Note that the hPLK1-siRNA used here was a 2'-OMe modification, which has been known not to trigger a type-I IFN response. The reason for which a mixture with an siRNA, but not the mRNA alone, was loaded in the lipid nanoparticle was to increase the amount of the lipid nanoparticles to be administered to an animal individual without changing the dosage of the mRNA, and thereby to increase the measuring sensitivity in the measurement of the lipid nanoparticle uptake efficiency.

The lipid solution was prepared by dissolving all lipid components for constituting the lipid nanoparticle and a fat-soluble fluorescent dye DiO in ethanol. DiO was added in an amount of 0.1 mol % in relation to all the lipid components. By including DiO in the lipid solution, a lipid nanoparticle modified with DiO was produced.

An appropriate amount of the nucleic acid-containing aqueous solution was aliquoted in a syringe of 2.5 mL volume, and an appropriate amount of the lipid solution was aliquoted in a syringe of 1 mL volume, each of which is connected to the lipid nanoparticle producing device via a syringe pump and introduced into the lipid nanoparticle producing device such that the ratio of the flow rate of the nucleic acid-containing aqueous solution to that of the lipid solution will be 3:1 (NIP ratio = 7.4 in the cases of A-11 composition hereinafter), and the total flow rate will be 500 μL/min.

The solution discharged from the dilution flow path of the lipid nanoparticle producing device was transferred onto a dialysis membrane ("Spectra/Por 4 dialysis membrane", MWCO: 12,000-14,000, Spectrum Laboratories Ltd.), the outer water phase was replaced by MES buffer (20 mM, pH 6.0), and dialyzed at 4 °C for at least 2 hours. Then, the outer water phase was replaced by PBS (-) and dialyzed again at 4 °C for at least 2 hours to remove ethanol and to substitute the aqueous solvent within the lipid nanoparticle with PBS. The solution collected after finishing dialysis was used as the lipid nanoparticle solution (LNP solution).

<Measurement of Average Particle Diameter and Zeta Potential of Lipid Nanoparticles>

For the lipid nanoparticles, the average particle diameter (number average, ζ average) in PBS (-) and the zeta potential in 10 mM HEPES buffer (pH 7.4) were measured using an analytic device "Zetasizer Nano ZS ZEN3600" (Malvern Panalytical Ltd.) that utilizes dynamic light scattering.

<RNA-Encapsulating Efficiency by Lipid Nanoparticles>

The RNA-encapsulation efficiency by the lipid nanoparticles was quantified by an assay using "Quant-iT® RiboGreen® RNA" (Thermo Fisher Scientific Inc.) which selectively causes an intercalation to an RNA and emits fluorescence (Ribogreen Assay).

A solution (Triton (+)) was prepared by mixing HEPES buffer (10 mM, pH 7.4), 10 % (w/v) Triton X-100, and RiboGreen® at 978.8 μL, 20 μL and 1.25 μL, respectively; another solution (Triton (-)) was prepared by mixing HEPES buffer (10 mM, pH 7.4) and RiboGreen® at 998.8 μL and 1.25 μL, respectively.

Moreover, as a sample for calibration curve, an RNA solution was prepared by diluting the RNA with HEPES buffer (10 mM, pH 7.4) such that the RNA is at 500 ng/mL. This RNA solution was diluted with HEPES buffer as appropriate to prepare an adequate dilution series.

The LNP solution was diluted with HEPES buffer (10 mM, pH 7.4) to be 500 ng/mL.

To a black 96-well plate for the measurement of the fluorescence, the sample for calibration curve and the diluted LNP solutions were added each in 100 μL/well, and further added was 100 μL/well of the solution comprising RiboGreen® (Triton (+) or Triton (-)). This plate was shaken on a shaking incubator ("SI-300", AS ONE Corp.) at 700 rpm for 30 seconds, and subsequently mounted on a microplate reader ("Enspire 2300 Multilabel Reader", PerkinElmer Co., Ltd.), and the fluorescence intensity of the solution in each well was measured (Excitation/Emission: 500/525 nm, Band width: 5 nm, Measurement time: 500 msec/well, Dynamic range: Autorange, Temperature: r.t.). The obtained fluorescence intensities were used to calculate the RNA-encapsulation efficiency according to the following equation. Measurements for the sample for calibration curve and each LNP sample were carried out in duplicate.

[RNA-encapsulation efficiency (%)] = ([RNA concentration in Triton(+)] - [RNA concentration in Triton (-)]) / [RNA concentration in Triton(+)] x 100

<Preparation of NLuc-mRNA>

NLuc-mRNA was prepared from pDNA comprising NLuc gene (pUC57-amp-T7-NLuc-IRES-RFP) by *in vitro* transcription.

First, the pDNA (pUC57-amp-T7-NLuc-IRES-RFP) was digested by a restriction enzyme EcoRV, and the DNA was purified from the digest by phenol-chloroform method, which is then dissolved in sterilized water to give a 1 μg/μL solution of linearized pDNA. Next, this linearized pDNA was used as the temperate to perform reactions of transcription and poly(A) addition. The reactions of transcription and poly(A) addition were carried out using a commercial kit ("mMESSAGE mMACHINE™ T7 Ultra Transcription Kit", Invitrogen) according to the protocol attached to the kit. Using the kit, an mRNA was synthesized *in vitro* in which a cap analogue (in which the 3'-OH group of m7G nucleotide was substituted by an $OCH_3$ group) was added to the 5'-terminal and a poly(A) was added to the 3'-terminal.

The mRNA obtained after the reaction was purified by phenol-chloroform, precipitated in isopropanol, collected and air dried, then redissolved in sterilized water. This redissolved RNA solution was purified using commercial kits for concentrating and purifying RNAs.

The RNA obtained by *in vitro* synthesis was subjected to an agarose gel electrophoresis to confirm the addition of 5'-terminal cap structure and 3'-terminal poly(A).

<Measurements of Nluc Expression Levels in Liver and Spleen>

The RNA-loaded, DiO-labelled lipid nanoparticles were administered to C57BL/6N mice via tail vein, and the mice were euthanatized to harvest the liver and spleen. The liver and spleen were weighed, then frozen and stored at -80 °C. A part of the spleen was saved before freezing/storing for use in analyses of splenic immunocytes, and the residual was frozen and stored.

To a sample tube containing the frozen/stored tissue and zirconia beads (diameter: 1.4 mm), 1 mL/tube of 1 x Passive Lysis Buffer was added, the tissue was milled using a bead-mill type cell disintegrator at 4 °C at 5,000 rpm for 40 seconds. The resulting debris was centrifuged at 4 °C at 13,000 rpm for 10 minutes, then the supernatant was collected as a sample for measurement.

The Nluc expression level was measured using a commercial luciferase assay system ("Nano-Glo® Luciferase Assay System", Promega Corp.). First, Nano-Glo® Luciferase Assay Substrate and Nano-Glo® Luciferase Assay Buffer were mixed at 50:1 to give Nano-Glo® Luciferase Assay Reagent. The sample for measurement 25 μL and Nano-Glo® Luciferase Assay Reagent 25 μL were mixed, incubated for 3 minutes, then the luminescence level of each sample was measured by a luminometer ("Luminescencer-PSN AB-2200" ATTO Corp.). The protein level in relation to the luciferase luminescence level was corrected between organs by a BCA (Bicinchoninic acid) assay. The BCA assay was carried out using a commercial BCA assay kit ("Pierce™ BCA protein assay", Pierce) using BSA (bovine serum albumin) as a protein for generating the calibration curve.

<Measurement of Cellular Uptake of Lipid Nanoparticles and Nluc Expression Levels in Splenic Immunocytes>

A part of the spleen was shredded by scissors, an appropriate amount of Hank's balanced salt solution (-) (HBSS (-)) was added to dissociate cells from the spleen. The resulting suspension was passed through a 40 μm cell strainer, and centrifuged at 4 °C at 400 g for 5 minutes. After removing the supernatant, the precipitate was resuspended by adding 1 mL of RBC lysis buffer and incubated for 3 minutes at room temperature. Then, the suspension was diluted 10-fold with HBSS (-), and centrifuged again at 4 °C at 400 g for 5 minutes to remove the supernatant. The resulting precipitate was resuspended with an appropriate amount of HBSS (-), and the number of cells was counted. The suspension was aliquoted in tubes of 1.5 mL volume at appropriate cell counts, and centrifuged at 4 °C at 400 g for 5 minutes. After removing the supernatant, the precipitate was blocked by adding a purified anti-mouse CD16/32 antibody (prepared at 10 μg/mL with FACS buffer) at 100 μL/1.0 $\times$ $10^6$ cells and incubating the mixture for 10 minutes at 4 °C while being tapped at 5-minute intervals.

After the blocking, the cells were stained with antibodies, using a PE-labelled anti-mouse F4/80 antibody, an APC-labelled anti-mouse CD11c antibody, a PerCP/Cyanine5.5-labelled anti-mouse I-A / I-E antibody, and a PE/Cy7-labelled anti-mouse CD19 antibody, and incubated for 30 minutes at 4 °C while being tapped at 5-minute intervals. After finishing the incubation, the cells were prepared in 1000 μL/tube with FACS buffer, and centrifuged at 4 °C at 400g for 5 minutes. After two more rounds of washing with 1 mL FACS buffer, the residue was used as a sample for FACS analysis.

FACS was carried out using a cell sorter ("SH800", Sony Corp.). To the sample, PI (propidium iodide) solution

was added at 6 μL/1 × $10^6$ cells. After about 15 minutes, dendritic cells (DC cells), macrophages (Mφ), and B cells were sorted to tubes of 1.5 mL volume containing 20 μL of 2 x Passive Lysis Buffer using the cell sorter such that the cell count is 3000 cells (about 20 μL) each. From the expression patterns of cell surface antigens, I-A/I-E-positive and CD11c-positive cells were sorted as dendritic cells, F4/80-positive cells as macrophages, and CD19-positive cells as B cells.

The correction of the fluorescence in the cell sorter was carried out using a commercial kit for fluorescence correction ("VersaComp Antibody Capture Bead Kit", Beckman Coulter Inc.). The preparation of the beads for correcting the leakage of the fluorescence in the cell sorter was carried out according to the protocol attached to the kit.

For each of the dendritic cell, B cell and macrophage fractioned by the cell sorter, the cellular uptake level of the lipid nanoparticles and the Nluc expression level were measured. The uptake level of the lipid nanoparticles was measured as an average value of the DiO fluorescence intensity in each cell. The measurement of the Nluc expression level in each cell was carried out using a commercial luciferase assay system ("Nano-Glo® Luciferase Assay System", Promega Corp.) as described above.

<Generation of Tumor-Bearing Mice>

In order to generate a tumor-bearing mouse, E.G7-OVA cell, a cultured cell line derived from OVA-expressing mouse malignant lymphoma, was transplanted subcutaneously to a mouse to generate a tumor-bearing mouse.

E.G7-OVA cells were cultured in RPMI-1640 medium at 37 °C under an environment of 5 volume % of $CO_2$. Counting of viable cells was done by trypan-blue staining.

E.G7-OVA cells were suspended in cold D-PBS(-) at 2 × $10^7$ cells/mL, and the cell suspension was transplanted subcutaneously to C57BL/6N mice (6-week old, female, supplied from Japan SLC,Inc.) who had been shaved on the right side of the abdomen, using a 26-gauge injection needle at 8 × $10^5$ cells/40 μL/mouse, while keeping the cells at 4 °C. It was ensured that it took 2 hours at most from the preparation of the cell suspension to the completion of the subcutaneous transplantation.

<Measurement of Tumor Volume>

The tumor volumes of the tumor-bearing mice were calculated every 3 days from 6 days after transplantation of E.G7-OVA cells using a digital caliper and according to the following equation.

$$[\text{Tumor Volume (mm}^3)] = [\text{Major Axis (mm)}] \times [\text{Miner Axis (mm)}] \times [\text{Miner Axis (mm)}] \times 0.52$$

The endpoint was set at tumor volume = 4000 $mm^3$, and mice who exceeded this were euthanized by cervical dislocation.

[Example 1]

A plan for definitive screening was carried out, which was focused on revealing the level of the main effect of each factor in the preparation of the lipid nanoparticle comprising a nucleic acid on the expression efficiency in splenic dendritic cells. The factors selected to be examined include the type and the content of the pH-sensitive cationic lipid, the content of the phospholipid, the content of the PEG lipid, and the NaCl concentration of the nucleic acid-containing aqueous solution, and a standard was designated to each of these factors.

As the lipid components that constitute the lipid nanoparticle, a pH-sensitive cationic lipid (CL), a phospholipid (PL), a cholesterol (chol), and a PEG lipid were used. As the pH-sensitive cationic lipid, CL4H6 or CL7H6 were used, and DOPE was used as the phospholipid. As the PEG lipid, PEG2k-DMG which is quickly released in blood, or PEG2k-DSG, which is not easy to be released and thus contributes to the retentivity in blood, was used. CL4H6 and CL7H6 used were those synthesized by methods described in Patent Literature 1. The pH-sensitive cationic lipid content (mol %), the phospholipid content (mol %), and the PEG lipid content (mol %) in relation to all lipid components for each examination plot were shown in Table 1. The cholesterol content (mol %) was the remainder of subtracting the contents of the pH-sensitive cationic lipid, phospholipid and PEG lipid from 100 mol %.

[Chem. 6]

CL4H6

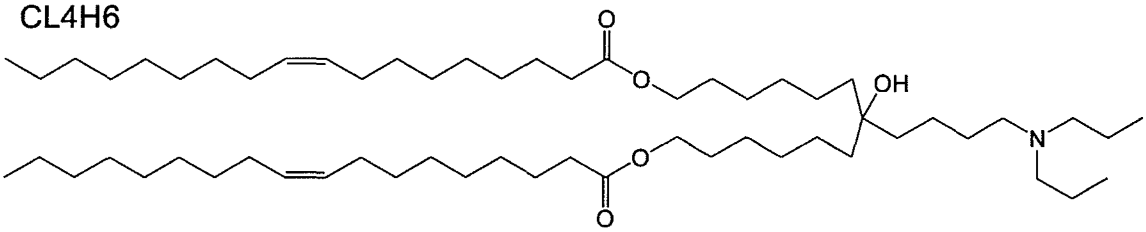

CL7H6

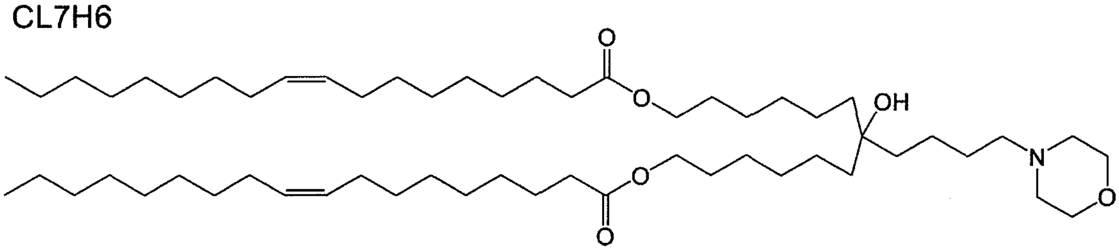

DOPE

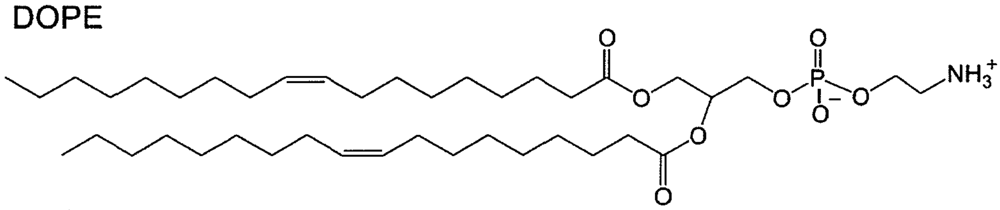

PEG2k-DMG

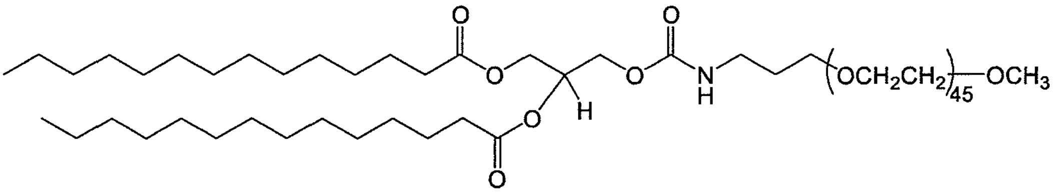

PEG2k-DSG

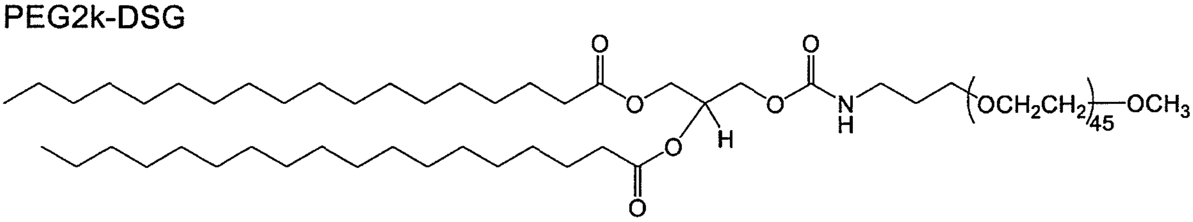

Cholesterol

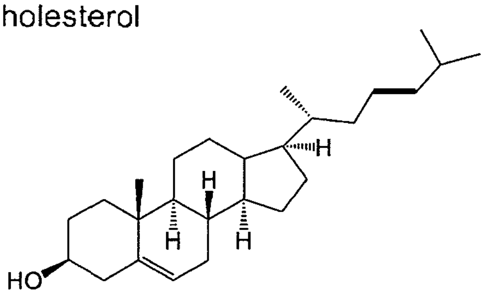

The experimental plan was made based on a data analyzing software "JMP®" (SAS Institute Japan Ltd.), extracting 14 types of compositions (A-1 to A-14) out of total 324 types ($3^4 \times 2^2$). Next, according to the lipid compositions described in Table 1, RNA-loaded lipid nanoparticles were prepared using nucleic acid-containing solutions prepared at NaCl concentrations described in Table 1. For each lipid nanoparticle prepared, the ζ average particle diameter (nm), the number average particle diameter (nm), PdI, and RNA-encapsulation efficiency (%) were measured. The measurements were made in duplicate (n=2) for a lipid nanoparticle sample of each examination plot. The results of measurements are shown in Table 2.

[Table 1]

| | CL [mol%] | PL [mol%] | PEG-lipid [mol%] | NaCl conc. [mM] | CL | PEG-lipid |
|---|---|---|---|---|---|---|
| A-1 | 40 | 40 | 0.5 | 0 | CL7H6 | PEG2k-DMG |
| A-2 | 50 | 10 | 0.5 | 0 | CL4H6 | PEG2k-DMG |
| A-3 | 60 | 10 | 1.0 | 0 | CL7H6 | PEG2k-DSG |
| A-4 | 60 | 40 | 1.5 | 0 | CL7H6 | PEG2k-DMG |
| A-5 | 40 | 25 | 1.5 | 0 | CL4H6 | PEG2k-DSG |
| A-6 | 60 | 40 | 0.5 | 200 | CL4H6 | PEG2k-DSG |
| A-7 | 50 | 25 | 1.0 | 200 | CL4H6 | PEG2k-DMG |
| A-8 | 40 | 10 | 1.5 | 200 | CL7H6 | PEG2k-DMG |
| A-9 | 50 | 25 | 1.0 | 200 | CL7H6 | PEG2k-DSG |
| A-10 | 40 | 40 | 1.0 | 400 | CL4H6 | PEG2k-DMG |
| A-11 | 60 | 10 | 1.5 | 400 | CL4H6 | PEG2k-DSG |
| A-12 | 50 | 40 | 1.5 | 400 | CL7H6 | PEG2k-DSG |
| A-13 | 60 | 25 | 0.5 | 400 | CL7H6 | PEG2k-DMG |
| A-14 | 40 | 10 | 0.5 | 400 | CL4H6 | PEG2k-DSG |

[Table 2]

| | ζ average particle diameter [nm] | Number average particle diameter [nm] | PdI | RNA-encapsulation efficiency [%] |
|---|---|---|---|---|
| A-1 | 112.2 | 69.7 | 0.195 | 90.25 |
| A-2 | 120.9 | 67.9 | 0.187 | 99.18 |
| A-3 | 103.9 | 64.8 | 0.151 | 90.99 |
| A-4 | 127.7 | 42.7 | 0.410 | 89.76 |
| A-5 | 87.7 | 42.8 | 0.295 | 99.19 |
| A-6 | 154.6 | 107.3 | 0.130 | 98.76 |
| A-7 | 137.7 | 95.4 | 0.123 | 99.03 |
| A-8 | 127.5 | 77.3 | 0.202 | 85.03 |
| A-9 | 171.3 | 86.1 | 0.153 | 87.99 |
| A-10 | 215.6 | 184.2 | 0.082 | 98.94 |
| A-11 | 546.6 | 489.8 | 0.193 | 89.21 |
| A-12 | 108.3 | 84.7 | 0.066 | 88.26 |
| A-13 | 500.8 | 439.8 | 0.116 | 91.15 |
| A-14 | 753.8 | 649.5 | 0.170 | 72.41 |

As shown in Table 2, the lipid nanoparticles prepared had the ζ average particle diameter in a range from about 80 to 750 nm. Moreover, for PdI, which is an indication of the uniformity of lipid nanoparticles, a majority of lipid nanoparticles indicate 0.2 or less. The RNA-encapsulation efficiency was 70 % or higher for all the lipid nanoparticles. By a statistical analysis testing the effect of each factor on the ζ average particle diameter, it was shown that the proportion of DOPE, the proportion of the PEG lipid, and the NaCl concentration at the time of preparing the lipid nanoparticle had influences on the ζ average particle diameter with statistical significance (Table 3, FIG. 2). In more detail, it became

clear that the ζ average particle diameter of the lipid nanoparticle was increased by decreasing the proportion of DOPE, by decreasing the proportion of the PEG lipid, and by increasing the NaCl concentration at the time of preparing the lipid nanoparticle.

[Table 3]

| | Testing of effects | | | | | |
|---|---|---|---|---|---|---|
| | Factors | Number of parameters | Degree of freedom | Sum of squares | $F_{value}$ | $p_{value}$ (Prob>F) |
| Main effect | PL [mol%] (10, 40) | 1 | 1 | 87291.65 | 74.0636 | <0.0001* |
| | PEG-lipid [mol%] (0.5, 1.5) | 1 | 1 | 41538.03 | 35.2434 | 0.0006* |
| | NaCl conc. [mM] (0, 400) | 1 | 1 | 247338.53 | 209.8572 | <0.0001* |
| Reciprocal action between 2 factors | PL[%]*NaCl conc. [mM] | 1 | 1 | 95451.36 | 80.9868 | <0.0001* |
| | PEG-lipid [mol%]* NaCl conc. [mM] | 1 | 1 | 14883.04 | 12.6277 | 0.0093* |
| | NaCl conc. [mM]* NaCl conc. [mM] | 1 | 1 | 41125.72 | 34.8936 | 0.0006* |

It was also shown that the proportion of the PEG lipid and the NaCl concentration at the time of preparing the lipid nanoparticle had an influence on the number average particle diameter with statistical significance. In more detail, it became clear that the number average particle diameter of the lipid nanoparticle is increased by decreasing the proportion of the PEG lipid, and by increasing the NaCl concentration at the time of preparing the lipid nanoparticle. Furthermore, it was shown that the proportion of the pH-sensitive cationic lipid, the proportion of DOPE, and the type of the pH-sensitive cationic lipid had an influence on the RNA-encapsulation efficiency with statistical significance. In more detail, the RNA-encapsulation efficiency of the lipid nanoparticle was increased by increasing the proportion of the pH-sensitive cationic lipid, by increasing the proportion of DOPE, and by using CL7H6 as the pH-sensitive cationic lipid.

The lipid nanoparticle prepared in each examination plot was administered to c57BL/6N mice (6-week old, female, supplied from Japan SLC,Inc.) via tail vein at 1.0 mg of RNA/kg. Twenty-four hours after administrating the lipid nanoparticle, the mice were euthanatized, and the Nluc expression level was measured in the liver and spleen. Moreover, the cellular uptake of the lipid nanoparticle and Nluc expression level in dendritic cells, B cells, and macrophages in the collected spleen were also measured.

The results of measuring the Nluc expression level per one gram of protein in the liver and spleen of the mice to whom the lipid nanoparticles of A-1 to A-14 were administered were shown in FIG. 3. The results of measuring the lipid nanoparticle uptake level (the average DiO fluorescence intensity in each cell) in the splenic immunocytes of the mice to whom the lipid nanoparticles of A-1 to A-14 were administered were shown in FIG. 4, and the results of measuring the Nluc expression level in FIG. 5, respectively.

The Nluc gene expression level in the dendritic cells was high by the lipid nanoparticles of A-6, A-10, A-11, A-13 and A-14, and, in particular, the lipid nanoparticle ofA-11 exhibited the highest gene expression (FIG. 5). On the other hand, the cellular uptake of the lipid nanoparticle was higher in the macrophages than in the dendritic cells, for lipid nanoparticles of any examination plot (FIG. 4). From these results, it was confirmed that the efficiency of the gene expression in relation to the amount of the lipid nanoparticles incorporated into the cells was higher in the dendritic cells as compared to in the macrophages. Moreover, little lipid nanoparticles were incorporated into the B cells (FIG. 4), nor any gene expression was confirmed (FIG. 5).

In entire spleen, the highest Nluc expression level was exhibited by the lipid nanoparticle of A-11, and the lipid nanoparticles ofA-2 and A-7 also exhibited high expressions (FIG. 3). Since the lipid nanoparticles ofA-2 and A-7 showed very little gene expression in the dendritic cells, it was speculated that a large part of these lipid nanoparticles was incorporated into other splenic cells than the dendritic cells (e.g., macrophages). From these results, it was shown that a high level gene expression in entire spleen does not necessarily result in a high gene expression in the dendritic cells.

As a result of the statistical analysis, it was shown that the proportion of the pH-sensitive cationic lipid, the proportion of DOPE, the type of the pH-sensitive cationic lipid and the type of the PEG lipid had an influence on the Nluc expression level in the spleen with statistical significance. Moreover, a lipid nanoparticle having a number average

particle diameter of 100 nm or greater exhibited a higher gene expression in dendritic cells as compared to a lipid nanoparticle with a number average particle diameter less than 100 nm, with statistical significance. Furthermore, in the context of the gene expression in splenic dendritic cells (FIG. 4), from the frequency of the standard for each of the PEG content, the NaCl concentration and the type of the pH-sensitive cationic lipid occurring in the lipid nanoparticles of the upper five examination plots and the lipid nanoparticles of the lower five examination plots, it was suggested that an increased PEG content, an increased NaCl concentration and the use of CL4H6 as the pH-sensitive cationic lipid are suitable for improving the efficiency of gene expression in the splenic dendritic cells.

[Example 2]

The lipid composition of the lipid nanoparticle was reexamined using "Taguchi Plan" (a partially enforced plan)in which the composition can be narrowed down by equally designating values for a factor to the specified area.

Based on the results of Example 1 and considering that the particle diameter contributes to the gene expression in dendritic cells, the NaCl concentration of the nucleic acid-containing aqueous solution at the time of preparing the lipid nanoparticle was set at 300 mM, so that the average particle diameter will be equal to or greater than 100 nm. Moreover, CL4H6 was used as the pH-sensitive cationic lipid in view of its main effect that will influence on the increase in the gene expression in the spleen. Other factors selected to be examined include the CL4H6 content, the phospholipid (DOPE) content, the type of the PEG lipid, and the PEG lipid content, and a standard was designated to each of these factors.

The experimental plan was made based on the data analyzing software "JMP®", extracting 8 types of compositions (B-1 to B-8) out of total 16 types ($2^4$). Next, according to the lipid compositions described in Table 4, RNA-loaded lipid nanoparticles were prepared using the nucleic acid-containing solutions prepared at NaCl concentration of 300 mM. For each lipid nanoparticle prepared, the ζ average particle diameter (nm), the number average particle diameter (nm), PdI, and RNA-encapsulation efficiency (%) were measured. The measurements were made in duplicate (n=2) for a lipid nanoparticle sample of each examination plot. The results of measurements are shown in Table 5.

[Table 4]

| | CL [mol%] | PL [mol%] | PEG-lipid [mol%] | PEG-lipid |
|---|---|---|---|---|
| B-1 | 50 | 10 | 0.75 | PEG2k-DMG |
| B-2 | 50 | 10 | 1.5 | PEG2k-DSG |
| B-3 | 50 | 20 | 0.75 | PEG2k-DSG |
| B-4 | 50 | 20 | 1.5 | PEG2k-DMG |
| B-5 | 60 | 10 | 0.75 | PEG2k-DSG |
| B-6 | 60 | 10 | 1.5 | PEG2k-DMG |
| B-7 | 60 | 20 | 0.75 | PEG2k-DMG |
| B-8 | 60 | 20 | 1.5 | PEG2k-DSG |

[Table 5]

| | ζ average particle diameter [nm] | Number average particle diameter [nm] | PdI | RNA-encapsulation efficiency [%] |
|---|---|---|---|---|
| B-1 | 327.4 | 300.4 | 0.135 | 91.47 |
| B-2 | 161.6 | 112.5 | 0.140 | 93.37 |
| B-3 | 254.5 | 227.4 | 0.068 | 99.66 |
| B-4 | 107.3 | 86.6 | 0.046 | 99.52 |
| B-5 | 423.7 | 396.3 | 0.093 | 93.08 |
| B-6 | 152.8 | 131.3 | 0.039 | 83.77 |
| B-7 | 286.3 | 245.0 | 0.064 | 99.42 |
| B-8 | 164.4 | 122.1 | 0.096 | 97.21 |

As shown in Table 5, the lipid nanoparticles of all examination plots had the ζ average particle diameter in a range between about 110 and 420 nm. The PdI was equal to or less than 0.15, and the RNA-encapsulation efficiency was equal to or greater than 80%, respectively, in all lipid nanoparticle. From the results of the statistical analysis, it was shown that the proportion of the PEG lipid had an influence on the number average particle diameter with statistical significance, and that the proportion of DOPE had an influence on the RNA-encapsulation efficiency with statistical significance. The results of the statistical analysis regarding the physical properties of the lipid nanoparticles were almost similar to the results of Example 1.

Subsequently, the lipid nanoparticles of Examination Plots B-1 to B-8 were administered to c57BL/6N mice via tail vein in a similar manner to Example 1, and the Nluc expression levels in the liver and spleen, as well as the cellular uptake of the lipid nanoparticles and the Nluc expression levels in the dendritic cells, B cells, and macrophages in the spleen were measured. The results of measuring the lipid nanoparticle uptake level (the average DiO fluorescence intensity in each cell) in splenic immunocytes of the mice to whom the lipid nanoparticles of B-1 to B-8 were administered were shown in FIG. 6, and the results of measuring the Nluc expression level in FIG. 7, respectively.

The Nluc gene expression level in the dendritic cell was high in the lipid nanoparticles of B-1, B-3, B-5 and B-7, and, in particular, the lipid nanoparticle of B-5 exhibits the highest gene expression (FIG. 7). On the other hand, the cellular uptake of the lipid nanoparticle was higher in the macrophages than in the dendritic cells, for lipid nanoparticles of any examination plot (FIG. 6). From these results, it was confirmed that the efficiency of the gene expression in relation to the amount of the lipid nanoparticles incorporated into the cells was higher in the dendritic cells as compared to in the macrophages.

As results of the statistical analysis, a high correlation was confirmed between the gene expression level in the dendritic cells and the particle diameter of the lipid nanoparticle. This correlation between these was higher than that in the experimental result of Example 1. It was speculated that this is because the composition of the lipid nanoparticle was examined in a narrower range in this example as compared to Example 1, which elevated the degree of the relative contribution of the particle diameter to the gene expression in the dendritic cell.

The results of the Nluc gene expression levels of the lipid nanoparticles of Examination Plots A-1 to A-14 in Example 1 and the lipid nanoparticles of Examination Plots B-1 to B-8 in splenic dendritic cells were summarized in FIG. 8. Among these lipid nanoparticles, the lipid nanoparticle of A-11 exhibited the highest Nluc gene expression level. Moreover, the particle diameter of the lipid nanoparticle, the cellular uptake by the dendritic cell, and the gene expression in the dendritic cell showed high correlationsrespectively. Therefore, the elevation of the gene expression in the dendritic cell associated with the increase in the particle diameter of the lipid nanoparticle is considered to be due to an increase in cellular uptake. Moreover, the gene expression in the spleen and the gene expression in the dendritic cell showed certain correlation in a similar manner to Example 1, though it was also shown that a lipid nanoparticle which exhibits a high gene expression in the spleen does not necessarily exhibit a high gene expression in the dendritic cell.

Furthermore, for these lipid nanoparticles, the relationship between the lipid nanoparticle uptake level by the dendritic cell (the average DiO fluorescence intensity) and the expression level of I-A / I-E, a dendritic cell maturation marker, was shown in FIG. 9(A), and the relationship between the Nluc gene expression level in the dendritic cell and the expression level of I-A / I-E is shown in FIG. 9(B), respectively. As a result, a high correlation was shown between the the lipid nanoparticle uptake by the dendritic cell and the expression level of I-A I-E (FIG. 9(A)). From these results, it became clear that the lipid nanoparticle of A-11 is efficiently incorporated into the splenic dendritic cell and thereby induces the maturation of the dendritic cell and at the same time accomplishes a high gene expression. It is expected that the lipid nanoparticle according to the present invention will exert a high performance as an mRNA vaccine, because it not only elicits an efficient gene expression in dendritic cells, but also elicits dendritic cell maturation.

[Example 3]

The reproducibility of the lipid nanoparticle of A-11 which exhibited the highest gene expression in splenic dendritic cells in Example 1 and 2, and the influence of the PEG lipid content were examined.

In specific, RNA-loaded lipid nanoparticles were prepared in a similar manner to A-11 of Example 1, according to the lipid compositions described in Table 6.

[Table 6]

| | Lipid compositions |
|---|---|
| A-11 | CL4H6 / DOPE / Chol / $_{m}PEG_{2k}$-DSG = 60 : 10 : 30 : 1.5 |
| A-15 | CL4H6 / DOPE / Chol / $_{m}PEG_{2k}$-DSG = 60 : 10 : 30 : 1.75 |
| A-16 | CL4H6 / DOPE / Chol / $_{m}PEG_{2k}$-DSG = 60 : 10 : 30 : 2.0 |

The prepared lipid nanoparticles were administered to c57BL/6N mice via tail vein in a similar manner to Example 1, and the level of cellular uptake of the lipid nanoparticle and the Nluc expression level in splenic dendritic cell were measured (n = 2 to 3). The results of measuring the lipid nanoparticle uptake level (the average DiO fluorescence intensity in each cell) were shown in FIG. 10 (A), and the results of measuring Nluc expression level were shown in FIG. 10 (B), respectively. As shown in FIG. 10, the level of cellular uptake of the lipid nanoparticle and the Nluc expression level in the splenic dendritic cell were both decreased in a PEG lipid content-dependent manner.

The prepared lipid nanoparticles were stored at 4 °C for 3 weeks, and the physical properties were measured over time. The results are shown in FIG. 11. FIG. 11 (A) shows the results of measuring the ζ average particle diameter (nm) of each lipid nanoparticle, FIG. 11 (B) shows the results of measuring the RNA-encapsulation efficiency (%) of each lipid nanoparticle. As a result, the RNA-encapsulation efficiency was on the same level irrespective of the PEG lipid content, though the ζ average particle diameter was decreased depending on the PEG lipid content. In either lipid nanoparticle, no particular changes in physical properties were observed after storing at 4 °C for 3 weeks, confirming a high stability in storage.

[Example 4]

It was expected that the lipid nanoparticle of Examination PlotA-11 in Example 1 and 3 can be applied as an mRNA vaccine that is capable of inducing an efficient gene expression in, and the maturation of, the splenic dendritic cells. Accordingly, its anti-tumor activity was evaluated using the lipid nanoparticle ofA-11 loaded with an mRNA encoding a tumor associated antigen.

In specific, first, an mRNA encoding OVA to which a 5'-terminal cap structure and a 3'-terminal poly(A) had been added (OVA-mRNA) was prepared by *in vitro* transcription in a similar manner to NLuc-mRNA using a pDNA comprising an ovalbumin (OVA) gene as a temperate. Next, a lipid nanoparticle in which OVA-mRNA had been loaded (mOVA-A11-LNP) was prepared in a similar manner to Examination Plot A-11 of Example 1, except that OVA-mRNA was used in place of NLuc-mRNA.

The prophylactic anti-tumor activity of mOVA-A11-LNP was evaluated as follows.

First, mOVA-A11-LNP was administered intravenously to mice twice every two week in 0.25 mg of OVA-mRNA per 1kg of the weight of the mouse (0.25 mg mRNA/kg), 0.5 mg mRNA/kg, or 0.75 mg mRNA/kg (n = 3 to 5 per each administered group). To the control mice, an equivalent amount of PBS was administered instead of mOVA-A11-LNP. After further 1 week, E.G7-OVA cells were subcutaneously transplanted on the right side of the abdomen, and the tumor volumes were measured every 3 days over time. The results of measuring the tumor volumes were shown in FIG. 12. As a result, in the mOVA-A11-LNP administered group, the engraftment of the tumor was completely prevented by any dosage equal to or greater than 0.25 mg mRNA/kg.

The therapeutic anti-tumor activity of mOVA-A11-LNP was evaluated as follows.

First, E.G7-OVA cells were subcutaneously transplanted on the right side of the abdomen of mice, and the tumor volumes were measured every 3 days over time. Seven, ten and fourteen days after transplantation, mOVA-A11-LNP was intravenously administered at 0.125 mg mRNA/kg, 0.25 mg mRNA/kg, or 0.5 mg mRNA/kg (n = 3 to 5 per each administered group). To the control mice, an equivalent amount of PBS was administered instead of mOVA-A11-LNP. The results of measuring the tumor volumes were shown in FIG. 13. In the figure, "**" indicates $p < 0.01$ in the nrANOVA followed by the SNK test. As a result, in the mOVA-A11-LNP administered group, a reduction in the tumor volume was observed by any dosage equal to or greater than 0.125 mg mRNA/kg. Among the twelve animals in total in the mOVA-A11-LNP administered group, the tumor disappeared completely in one out of four animals in the 0.25 mg mRNA/kg administered group and in one out of three animals in the 0.5 mg mRNA/kg administered group.

For the two mice in which the tumors were ameliorated by complete disappearance, E.G7-OVA cells were subcutaneously transplanted again 68 days after the subcutaneous transplantation of E.G7-OVA cells. Seven and ten days after transplantation, mOVA-A11-LNP was administered in the same dosage as the first time. For the PBS-administered control group and the two mice relieved from the first subcutaneous transplantation, the results of measuring the tumor volume were shown in FIG. 14. As a result, even after the re-transplantation of E.G7-OVA cells, mOVA-A11-LNP completely suppressed the tumor engraftment. As a reason for this, it can be considered that, in the mice administered mOVA-A11-LNP, there remained the memory T cells who had experienced an antigen-stimulation by OVA expressed in the splenic dendritic cell by mOVA-A11-LNP by which the anti-tumor activity had been persisted.

Moreover, since no mOVA-A11-LNP dosage-dependent anti-tumor activity was confirmed, it can be considered highly likely that a single dosage of 0.125 mg mRNA/kg has reached a saturation in the context of evaluating an anti-tumor activity. Therefore, an examination was conducted for an adequate dosage in evaluating the therapeutic anti-tumor activity of mOVA-A11-LNP. In specific, 8 and 11 days after subcutaneous transplantation of E.G7-OVA cells, mOVA-A11-LNP was administered via tail vein at a single dosage of 0.05 mg mRNA/kg, 0.015 mg mRNA/kg, 0.005 mg mRNA/kg, or 0.0015 mg mRNA/kg. The results of measuring the tumor volumes in each administered group were shown in FIG. 15. As a result, mOVA-A11-LNP exhibited a therapeutic anti-tumor activity when the dosage was equal to or

greater than 0.015 mg mRNA/kg.

[Example 5]

In order to investigate the utility of the lipid nanoparticle of Examination Plot A-11 of Example 1, a comparison was made in the gene expression with two mRNA delivery systems that have already been reported. One of the two mRNA delivery systems for comparison was RNA-LPX (Non-Patent Literatures 2 and 3, BioNTech SE) which is now under Phase I Clinical Trial as an mRNA cancer vaccine for melanoma, etc. The other was MC3-LNP, which is an equivalent preparation to Onpattro® (Alnylam Pharmaceutical Inc.), which is a lipid nanoparticle for RNA delivery with clinical achievements. Since MC3-LNP also exhibits certain efficiency as an mRNA delivery system, several examples of its usage as a comparative preparation have been reported (Non-Patent Literature 7).

The NLuc-mRNA loaded RNA-LPX (mNLuc-RNA-LPX) was prepared as follows.

First, DOTMA/DOPE (1:1 molar ratio, total volume 400 μL) was dissolved in ethanol such that the total lipid concentration is 10 mM, which is added to a glass test tube, and the solvent was removed using an evaporator. Next, 800 μL of D-PBS(-) was added to the test tube, which was left hydrated for two minutes. This was then stirred by a vortex mixer, and subsequently left still for five minutes. The test tube was subjected to ultrasonication using a bath-type sonicator (about 30 seconds), and the prepared liposome solution was diluted 5.92-fold with D-PBS(-) (the total lipid concentration = 0.845 mM). The solution was diluted with D-PBS(-) to 0.2 mg mRNA/mL, and to 100 μL of this solution, 100 μL of the liposome solution was added while stirring using a vortex mixer (NIP = 1.3/2).

The NLuc-mRNA loaded MC3-LNP (mNLuc-MC3-LNP) was prepared in a similar manner to Examination PlotA-11, except that the lipid composition was MC3/DSPC/Chol/PEG2k-DMG (50/10/40/1.5 mol%) and that no NaCl was added in the nucleic acid-containing aqueous solution.

Each of the prepared lipid nanoparticles was administered to mice via tail vein in a similar manner to Example 1 except that the dosage was 0.5 mg mRNA/kg, and the mice were euthanatized 24 hours after administration. The liver, spleen, lung, kidney and the inguinal lymph node were collected, and the Nluc expression levels were measured. The results of the measurements were shown in FIG. 16. As a result, the gene expression of mNLuc-MC3-LNP was shown high in the order of: the liver, spleen, inguinal lymph node, kidney, and lung, exhibiting a gene expression of about 16-fold in liver as compared to in spleen. The result was reasonable because mNLuc-MC3-LNP is a preparation that is capable of efficiently delivering a nucleic acid to hepatic parenchymal cells. mNLuc-RNA-LPX exhibited a high gene expression in the order of: spleen, inguinal lymph node, lung, liver, and kidney, and exhibited about 270-fold higher gene expression in the spleen as compared to in the inguinal lymph node. This was a reasonable result because RNA-LPX is a preparation with a very high selectivity for spleen in terms of gene expression. On the contrary, the lipid nanoparticle of A-11 exhibited, as compared to mNLuc-MC3-LNP and mNLuc-RNA-LPX, about 13-fold and about 5-fold gene expression in the spleen, and exhibited about 5-fold and about 100-fold gene expression in the inguinal lymph node, respectively. From these results, it was shown that the lipid nanoparticle of A-11 is more effective as a carrier for gene expression in lymphatic tissues than the two preparations with clinical achievements.

Next, the gene expression efficiencies of the lipid nanoparticles in the splenic dendritic cell were compared.

First, an mRNA encoding OVA to which a 5'-terminal cap structure and a 3'-terminal poly(A) had been added (EGFP-mRNA) was prepared by *in vitro* transcription in a similar manner to NLuc-mRNA using a pDNA comprising a gene of a fluorescent protein EGFP as the temperate. A lipid nanoparticle in which EGFP-mRNA had been loaded (mEGFP-A11-LNP) was prepared in a similar manner to Examination Plot A-11, except that EGFP-mRNA was loaded in place of NLuc-mRNA.

Furthermore, RNA-LPX in which EGFP-mRNA had been loaded (mEGFP-RNA-LPX) was prepared in a similar manner to the preparation of mNLuc-RNA-LPX, and MC3-LNP in which EGFP-mRNA had been loaded (mEGFP-MC3-LNP) was prepared in a similar manner to the preparation of mNLuc-MC3-LNP, except that EGFP-mRNA was loaded in place of NLuc-mRNA.

Each of the prepared lipid nanoparticles was administered to mice via tail vein in a similar manner to Example 1 except that the dosage was 0.5 mg mRNA/kg, and the mice were euthanatized 24 hours after administration. The spleen was collected, and the splenic dendritic cells were sorted by FACS. The proportions of EGFP-positive cells (%) in relation to the entire splenic dendritic cells are shown in FIG. 17(A), and the results of measuring the average EGFP fluorescence intensities in the splenic dendritic cells are shown in FIG. 17(B), respectively. As a result, the proportion of EGFP-positive cells in the mEGFP-A11-LNP administered group was about 9 %, which was about 5-fold and about 10-fold higher as compared to the mEGFP-MC3-LNP administered group and the mEGFP-RNA-LPX administered group, respectively.

At the same time, the expression level of I-A / I-E, which is a marker for the maturation of the splenic dendritic cell, was measured in a similar manner to Example 1. The results of the measurements are shown in FIG. 17(C). As a result, the mEGFP-A11-LNP administered group exhibited a significantly higher I-A / I-E expression level. The results above showed that mEGFP-A11-LNP exhibited a higher gene expression in the dendritic cells as compared to the two

preparations with clinical achievements, and at the same time it is capable of efficiently inducing the maturation of the dendritic cells.

[Example 6]

For mOVA-A11-LNP prepared in Example 4, RNA-LPX and MC3-LNP, their anti-tumor activities were compared.
An OVA-mRNA-loaded RNA-LPX (mOVA-RNA-LPX) was prepared in a similar manner to the preparation of mNLuc-RNA-LPX, except that the OVA-mRNA used in Example 4 was loaded in place of NLuc-mRNA.
Similarly, an OVA-mRNA-loaded MC3-LNP (mOVA-MC3-LNP) was prepared in a similar manner to the preparation of mNLuc-MC3-LNP, except that the OVA-mRNA was loaded in place of NLuc-mRNA.
Furthermore, a lipid nanoparticle in which an OVA-mRNA had been loaded (mOVA-B8-LNP) was prepared in a similar manner to the examination plot B-8 in Example 2, except that the OVA-mRNA was loaded in place of NLuc-mRNA. In Example 2, the Nluc expression level by the lipid nanoparticle of Examination Plot B-8 was equivalent as compared to the lipid nanoparticle of Examination Plot A-11 in entire the spleen, though it was lower in the splenic dendritic cell.
The therapeutic anti-tumor activities of mOVA-A11-LNP, mEGFP-A11-LNP, mOVA-B8-LNP, mOVA-RNA-LPX, and mOVA-MC3-LNP were evaluated using tumor-bearing mice to whom E.G7-OVA cells had been subcutaneously transplanted.
In specific, E.G7-OVA cells were subcutaneously transplanted to mice on the right side of the abdomen of mice, and the tumor volumes were measured every 3 days over time. Seven and ten days after the transplantation, the lipid nanoparticle was administered intravenously at 0.03 mg mRNA/kg (n = 5 per each administered group). To the control mice, an equivalent amount of PBS was administered instead of the lipid nanoparticle. The results of measuring the tumor volumes were shown in FIG. 18. As a result, a reduction in the tumor volume in the therapy duration was confirmed only in mOVA-A11-LNP administered group. No reduction in the tumor volume was confirmed in the mOVA-RNA-LPX administered group and the mOVA-MC3-LNP administered group. Therefore, it became clear that mOVA-A11-LNP exhibits an excellent anti-tumor therapeutic activity as compared to the two preparations. Furthermore, no reduction in the tumor volume was confirmed even in the mOVA-B8-LNP administered group, indicating a possibility that the gene expression at immunocyte level is an important indicator of the anti-tumor activity, but not the gene expression in entire spleen.

REFERENCE SIGNS LIST

10...First introduction path, 20...Second introduction path, 30...Dilution flow path, 31 ...Junction, 40...tructural element, 50...Bent flow path part.

## Claims

1. A lipid nanoparticle comprising a pH-sensitive cationic lipid and a polyalkylene glycol-modified lipid, wherein:

    the proportion of the pH-sensitive cationic lipid content to the total amount of the lipids constituting the lipid nanoparticle is from 40 to 70 mol %; and
    the proportion of the polyalkylene glycol-modified lipid content to the total amount of the lipids constituting the lipid nanoparticle is from 0.5 to 1.75 mol %;
    and wherein the lipid nanoparticle has a number average particle diameter equal to or greater than 150 nm.

2. The lipid nanoparticle according to Claim 1, having a number average particle diameter equal to or smaller than 600 nm.

3. The lipid nanoparticle according to Claim 1 or 2, wherein the pH-sensitive cationic lipid is represented by the following general formula (I-1):

[Chem. 1]

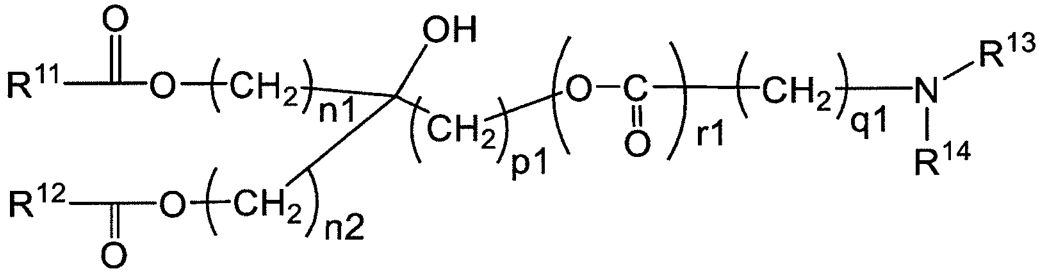

(I-1)

[in the formula (I-1), $R^{11}$ and $R^{12}$ are each independently a straight-chain $C_{10-14}$ alkyl group, a straight-chain $C_{10-20}$ alkenyl group having one or two unsaturated bond(s), or $-CH(R^{15})(R^{16})$($R^{15}$ and $R^{16}$ are each independently a straight-chain $C_{5-10}$ alkyl group); n1 and n2 each independently denotes an integer from 6 to 10; p1 and q1 denote integers that are equal to or greater than 0 and satisfy p1 + q1 = from 3 to 8; r1 denotes 0 or 1; $R^{13}$ and $R^{14}$ are each independently a straight-chain $C_{1-3}$ alkyl group or an aryl $C_{1-3}$ alkyl group, or $R^{13}$ and $R^{14}$ are joined together to form a pyrrolidine ring, a piperidine ring, a morpholine ring, or a piperazine ring in which a nitrogen atom may be substituted with a $C_{1-3}$ alkyl group].

**4.** The lipid nanoparticle according to any one of Claims 1 to 3, further comprising one or more selected from a group consisting of sterols and phospholipids.

**5.** The lipid nanoparticle according to any one of Claims 1 to 4, comprising a nucleic acid.

**6.** The lipid nanoparticle according to Claim 5, wherein the nucleic acid is an siRNA.

**7.** The lipid nanoparticle according to Claim 5, wherein the nucleic acid is an mRNA or a plasmid DNA.

**8.** The lipid nanoparticle according to any one of Claims 5 to 7, wherein the nucleic acid is a gene that is to be expressed in a splenic dendritic cell.

**9.** A pharmaceutical composition, wherein the lipid nanoparticle according to any one of Claims 1 to 8 is an active ingredient.

**10.** The pharmaceutical composition according to Claim 9 for use in cancer vaccine therapy.

**11.** The pharmaceutical composition according to Claim 9 for use in immunoactivation.

**12.** A method of expressing an exogenous gene comprising administrating the lipid nanoparticle according to any one of Claims 5 to 8 in which an exogenous gene of interest to be expressed in a splenic dendritic cell is encapsulated to a subject animal (excluding human), and expressing the exogenous gene in a splenic dendritic cell of the subject animal.

**13.** A method for producing the lipid nanoparticle according to any one of Claims 5 to 8 using a flow path structure, having

a forming step in which the lipid nanoparticle is formed in the flow path structure from a lipid solution in which all lipid components for constituting the lipid nanoparticle have been dissolved in ethanol and an aqueous solution comprising the nucleic acid; and
a dialysis step in which a solution comprising the lipid nanoparticle obtained in the forming step is dialyzed in a buffer solution at pH 6.8 to 7.6,

wherein:

the flow path structure forms one dilution flow path when a first introduction path for introducing a first fluid and a second introduction path for introducing a second fluid each independently has a specified length, and join together;

the dilution flow path has a flow path part that is two-dimensionally bent at least in a part thereof;

designating the axis direction of the dilution flow path upstream to the bent flow path part toward its extension direction to be the X direction, and designating the width direction of the dilution flow path that perpendicularly intersects this X direction to be the Y direction, and designating the width of the dilution flow path upstream to the bent flow path part to be $y_0$, the bent flow path part is formed by providing at least two or more structural elements with specified intervals $d_1$, $d_2$, ..., wherein the two or more structural elements protrude alternately from the both side faces of the dilution flow path opposing in the Y direction toward the center of the flow path, approximately in the Y direction (approximately in + Y direction and approximately in - Y direction), wherein the two or more structural elements have specified heights $h_1$, $h_2$, ... that are equal to or greater than 1/2 $y_0$ and smaller than 1 $y_0$, and have specified widths $x_1$, $x_2$, ..., in the X direction, and thereby define the width of the dilution flow path;

the lipid solution is introduced from the first introduction path and the aqueous solution comprising the nucleic acid is introduced from the second introduction path, respectively, and

the aqueous solution comprising the nucleic acid comprises the sodium chloride at a concentration equal to or greater than 280 mM, and the aqueous solution comprising the nucleic acid is acidic.

**14.** The method for producing a lipid nanoparticle according to Claim 13, wherein the sodium chloride concentration in the aqueous solution comprising the nucleic acid is equal to or less than 500 mM.

**15.** A kit for use in the method for producing a lipid nanoparticle according to Claim 13 or 14, comprising

a dry matter of a lipid composition comprising all lipid components for constituting the lipid nanoparticle; and

a dry matter comprising sodium chloride for being dissolved in water for preparation of an aqueous solution in which the sodium chloride concentration is equal to or greater than 280 mM.

**16.** The kit for use in the method for producing a lipid nanoparticle according to Claim 15, further comprising one or more selected from the group consisting of ethanol, phosphate buffered saline and a nucleic acid.

[FIG. 1]

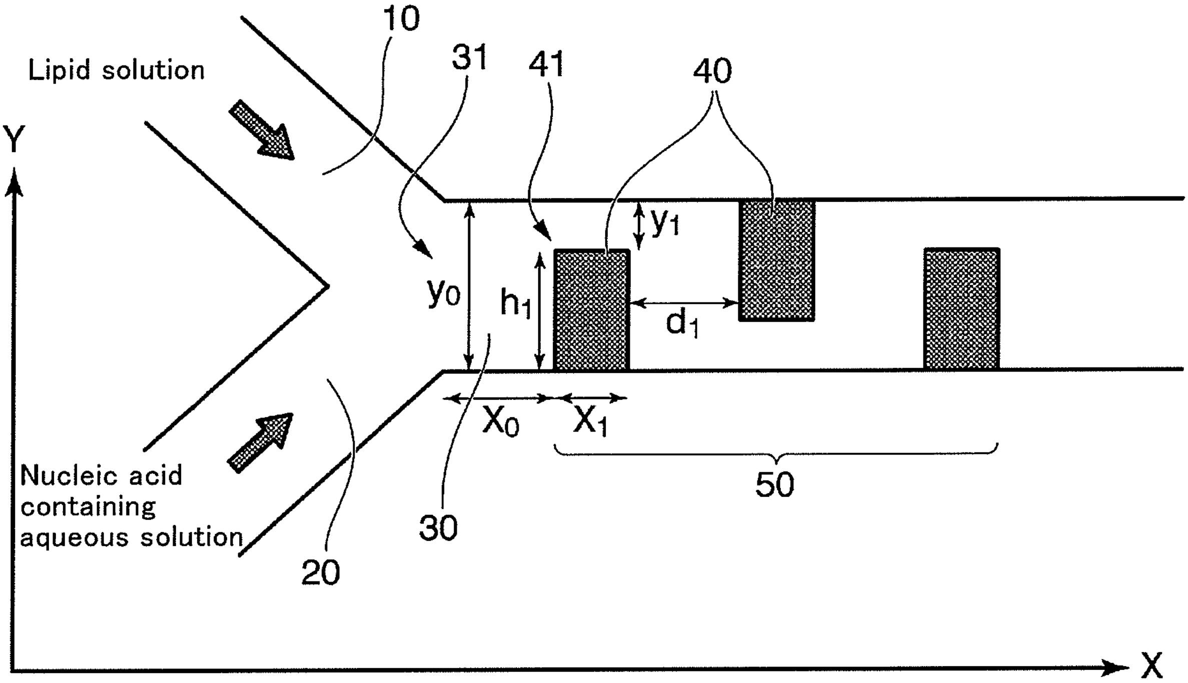
Lipid solution
Nucleic acid containing aqueous solution
Y
X
10
31
41
40
20
30
50
y0
y1
h1
d1
X0
X1

[FIG. 2]

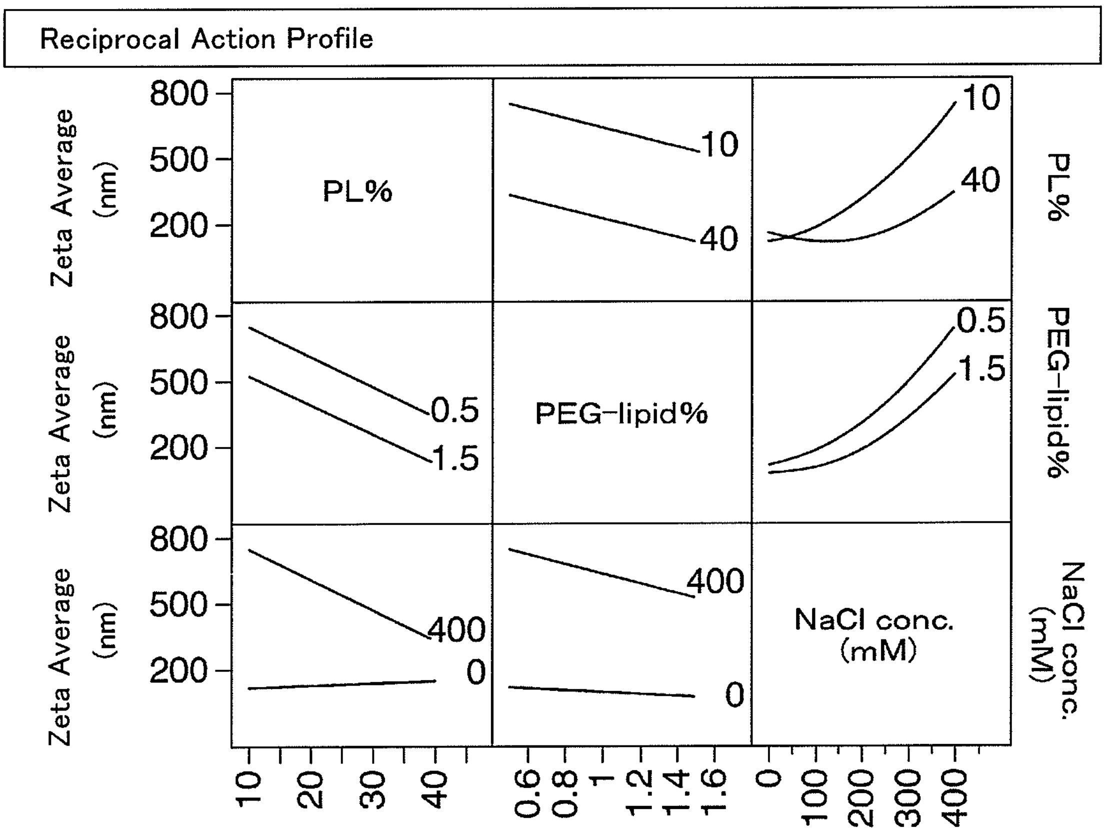
Reciprocal Action Profile
Zeta Average (nm)
800
500
200
PL%
PEG-lipid%
NaCl conc. (mM)
10
40
0.5
1.5
400
0
10 20 30 40
0.6 0.8 1 1.2 1.4 1.6
0 100 200 300 400

[FIG. 3]

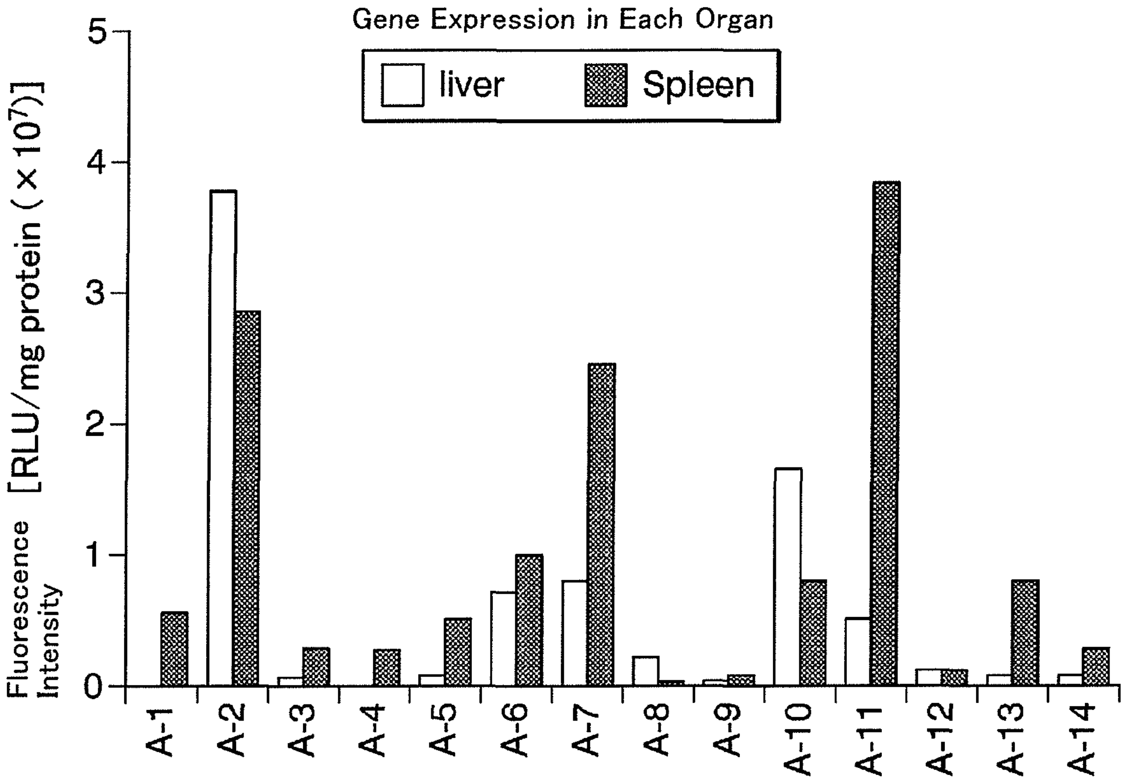
Gene Expression in Each Organ
liver
Spleen
Fluorescence Intensity [RLU/mg protein (×10^7)]
5
4
3
2
1
0
A-1
A-2
A-3
A-4
A-5
A-6
A-7
A-8
A-9
A-10
A-11
A-12
A-13
A-14

[FIG. 4]

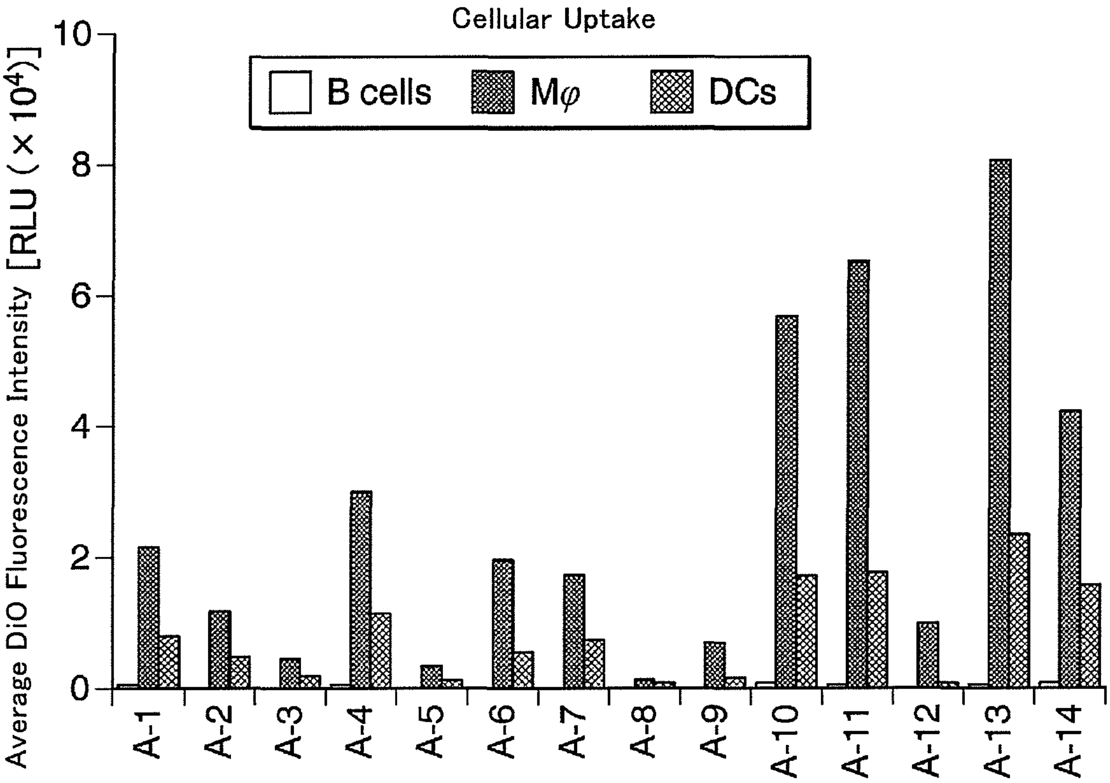
Cellular Uptake
B cells
Mφ
DCs
Average DiO Fluorescence Intensity [RLU (×10^4)]
10
8
6
4
2
0
A-1
A-2
A-3
A-4
A-5
A-6
A-7
A-8
A-9
A-10
A-11
A-12
A-13
A-14

[FIG. 5]

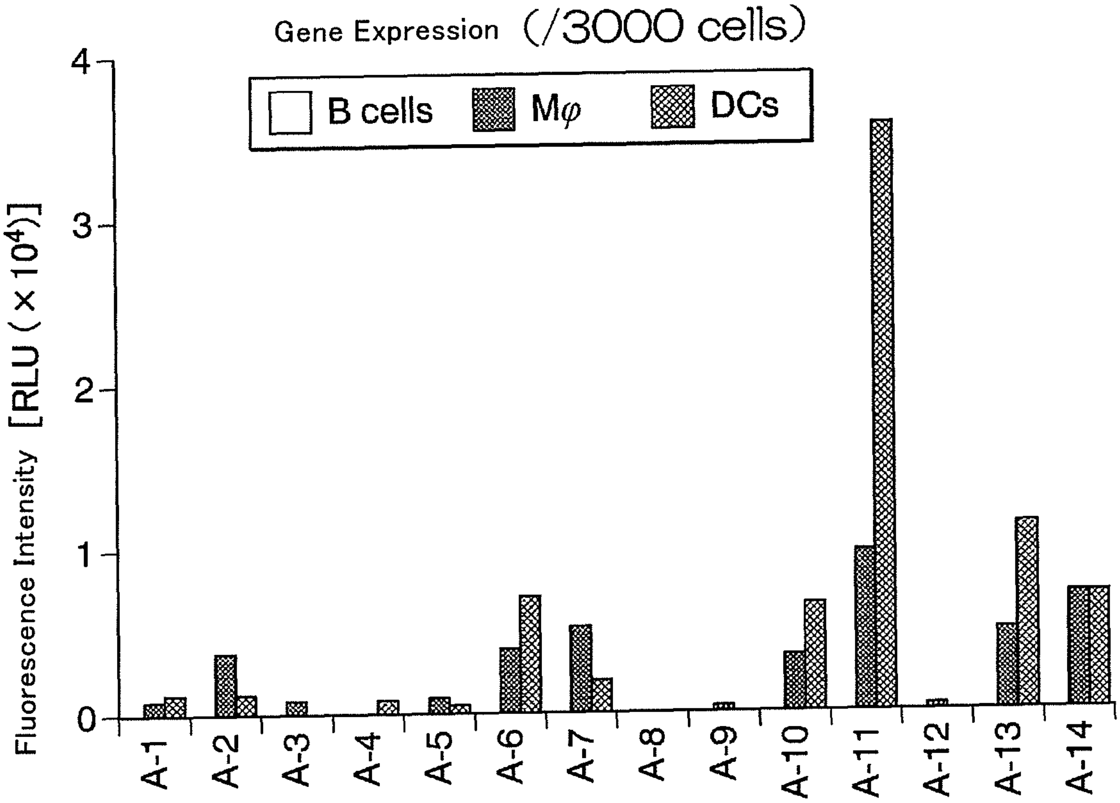
Gene Expression (/3000 cells)
B cells
Mφ
DCs
Fluorescence Intensity [RLU (×10^4)]
4
3
2
1
0
A-1
A-2
A-3
A-4
A-5
A-6
A-7
A-8
A-9
A-10
A-11
A-12
A-13
A-14

[FIG. 6]

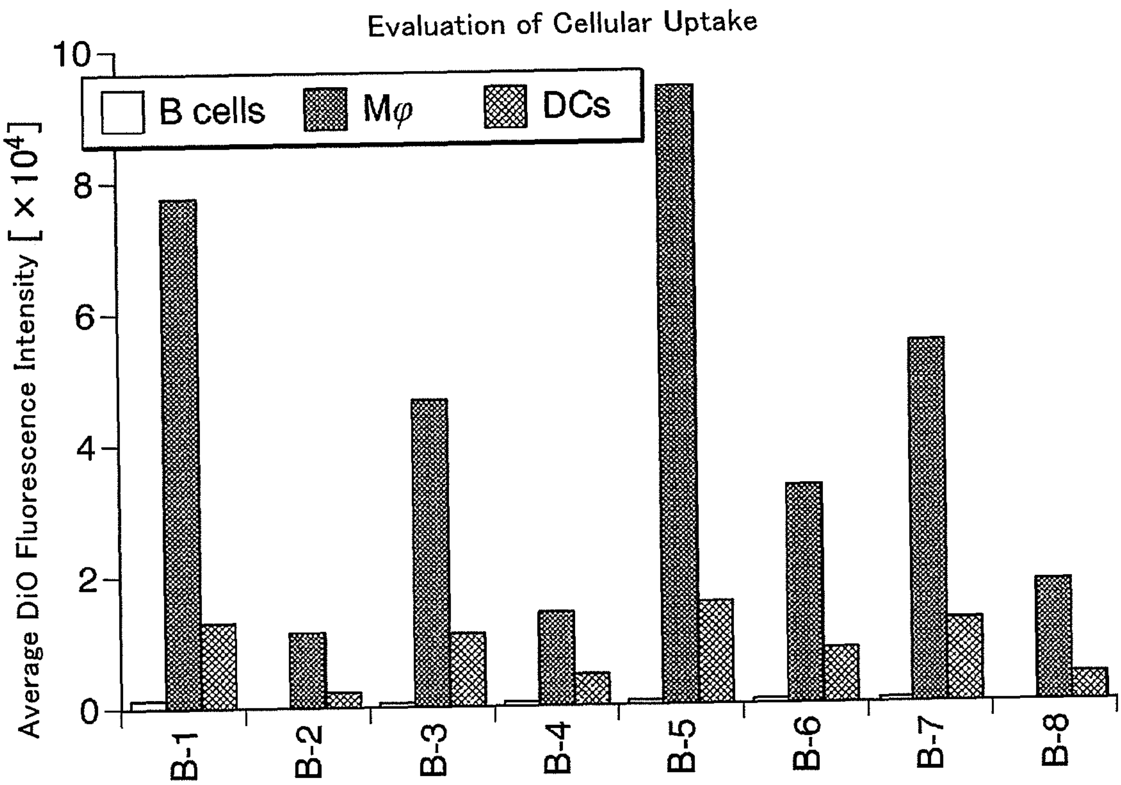
Evaluation of Cellular Uptake
B cells
Mφ
DCs
Average DiO Fluorescence Intensity [×10^4]
10
8
6
4
2
0
B-1
B-2
B-3
B-4
B-5
B-6
B-7
B-8

[FIG. 7]

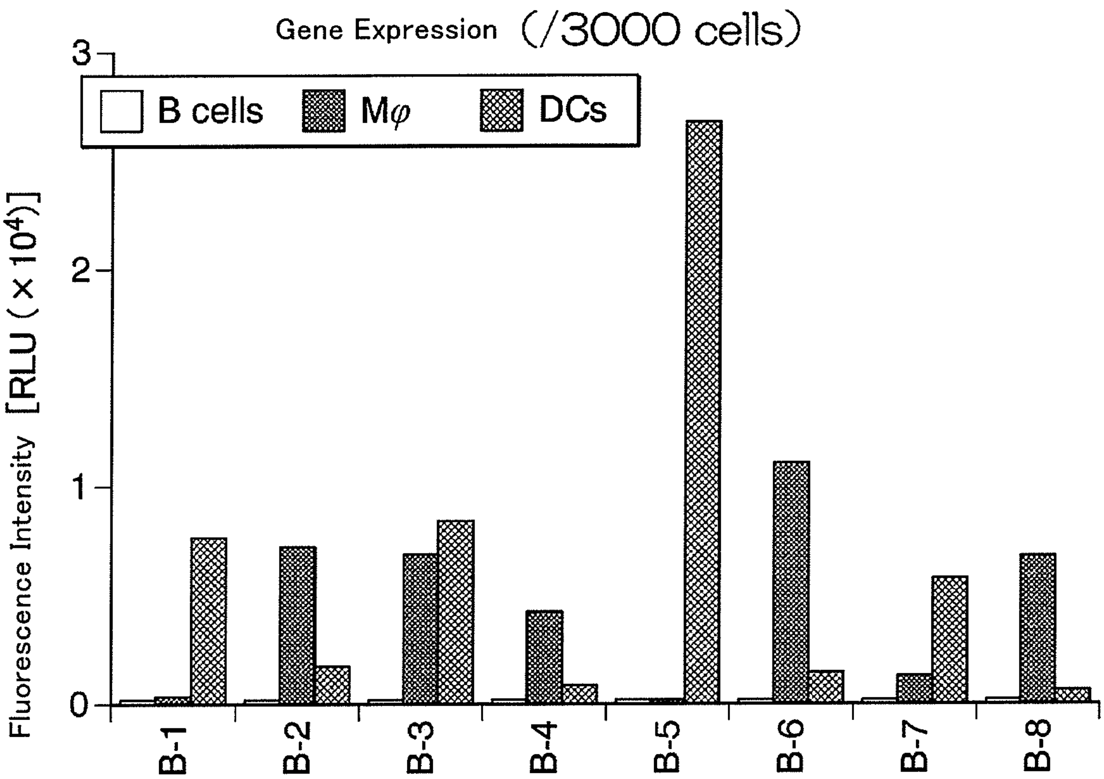
Gene Expression (/3000 cells)
B cells
Mφ
DCs
Fluorescence Intensity [RLU (×10⁴)]
0
1
2
3
B-1
B-2
B-3
B-4
B-5
B-6
B-7
B-8

[FIG. 8]

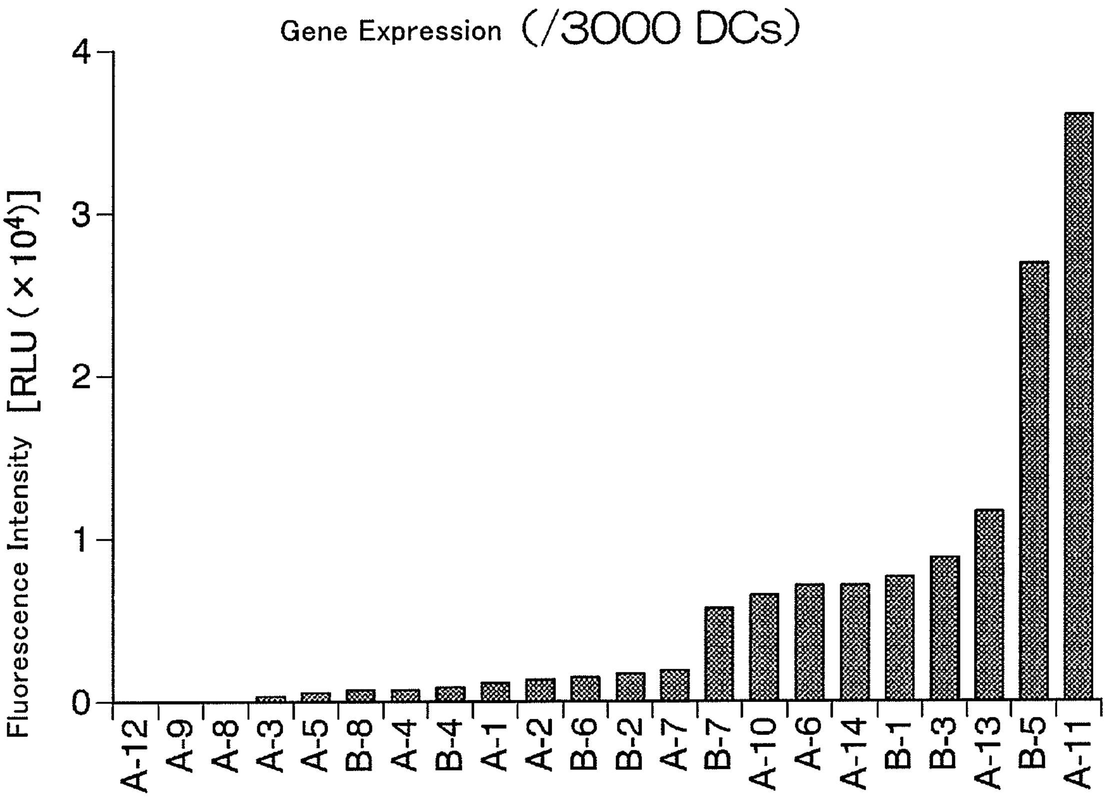
Gene Expression (/3000 DCs)
Fluorescence Intensity [RLU (×10⁴)]
0
1
2
3
4
A-12
A-9
A-8
A-3
A-5
B-8
A-4
B-4
A-1
A-2
B-6
B-2
A-7
B-7
A-10
A-6
A-14
B-1
B-3
A-13
B-5
A-11

[FIG. 9]

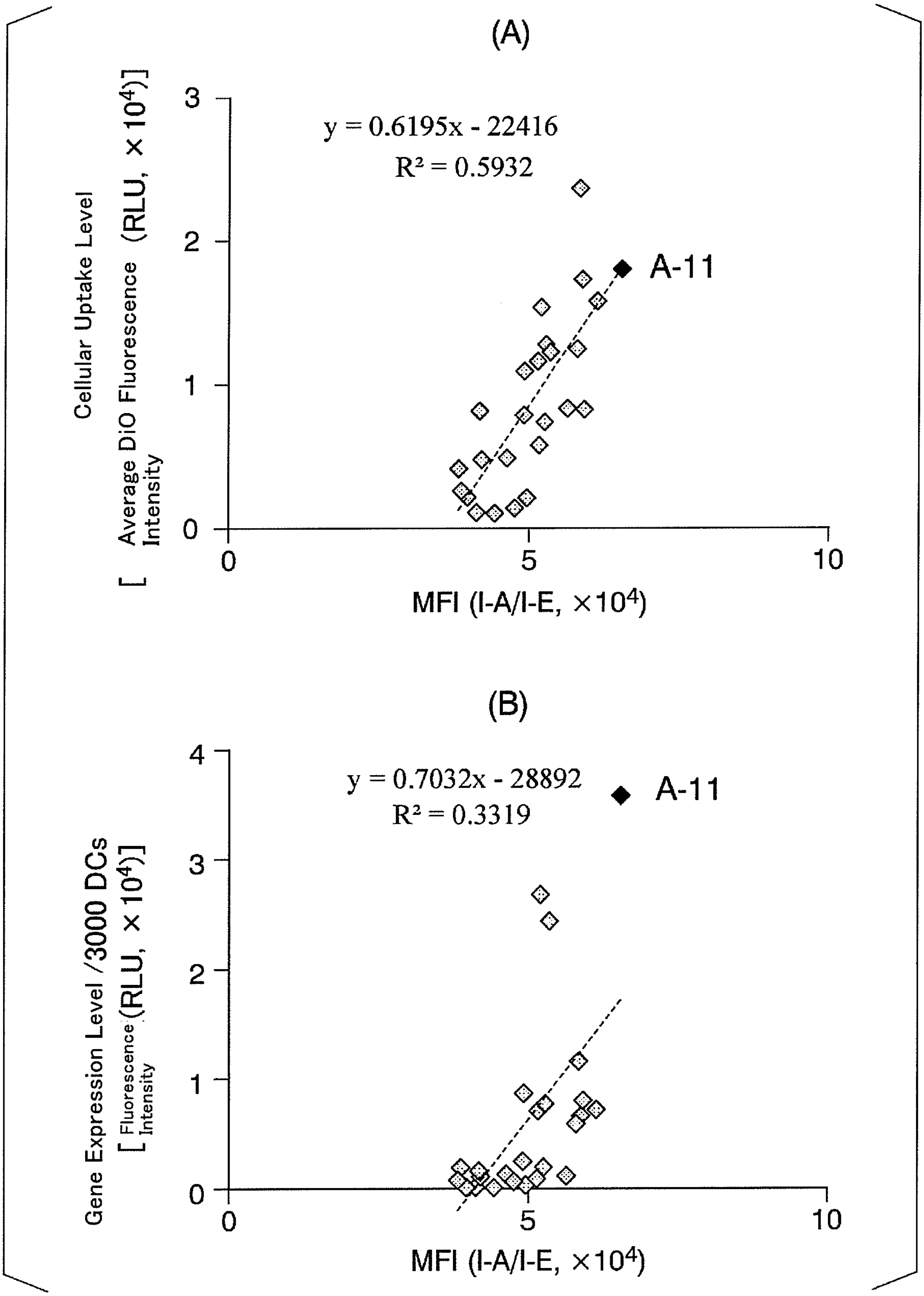
(A)
y = 0.6195x - 22416
R² = 0.5932
A-11
Cellular Uptake Level
[Average DiO Fluorescence Intensity (RLU, ×10⁴)]
MFI (I-A/I-E, ×10⁴)
(B)
y = 0.7032x - 28892
R² = 0.3319
A-11
Gene Expression Level /3000 DCs
[Fluorescence Intensity (RLU, ×10⁴)]
MFI (I-A/I-E, ×10⁴)

[FIG. 10]

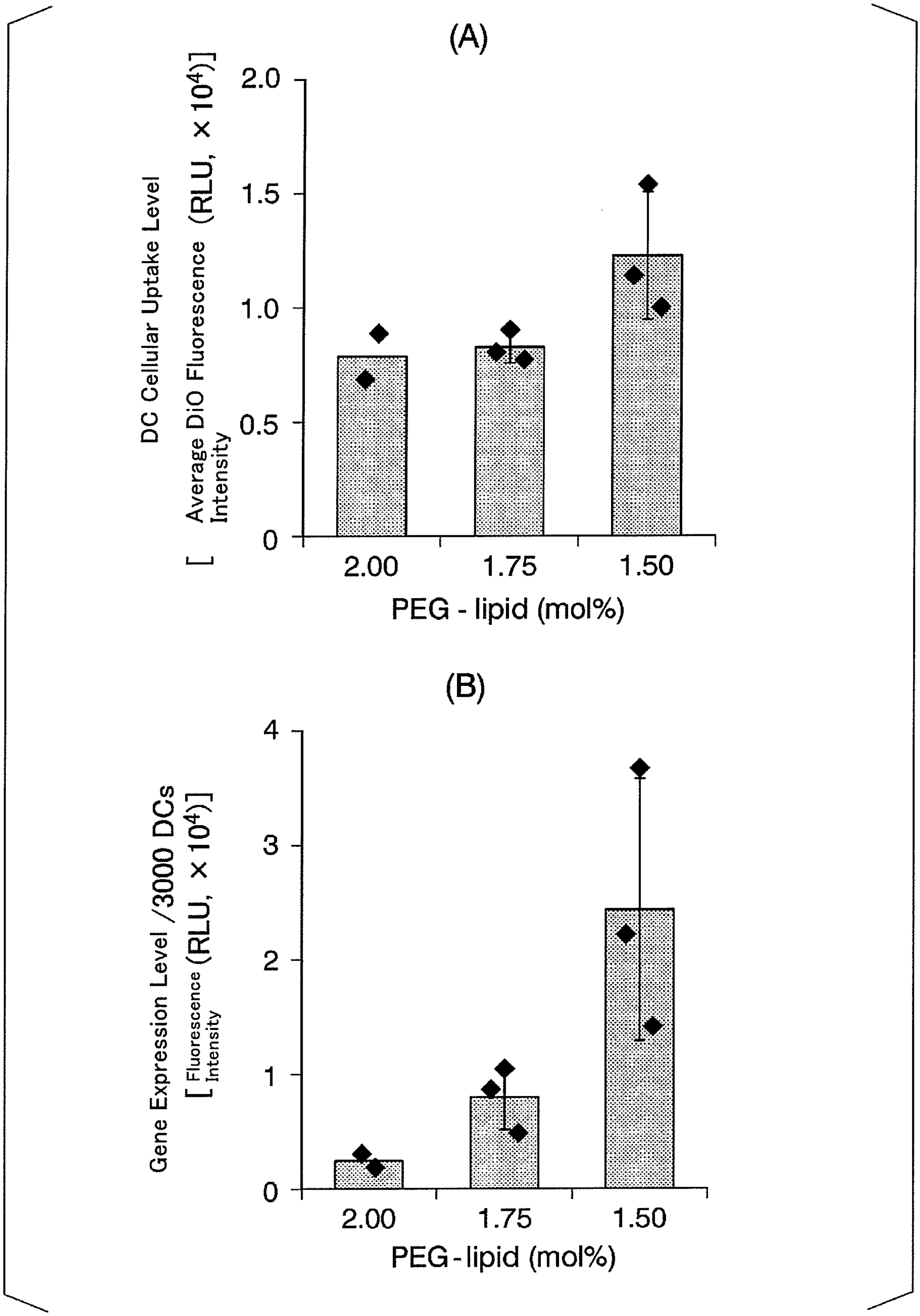
(A)
DC Cellular Uptake Level
[Average DiO Fluorescence Intensity (RLU, ×10^4)]
2.0
1.5
1.0
0.5
0
2.00
1.75
1.50
PEG - lipid (mol%)
(B)
Gene Expression Level /3000 DCs
[Fluorescence Intensity (RLU, ×10^4)]
4
3
2
1
0
2.00
1.75
1.50
PEG-lipid (mol%)

[FIG. 11]

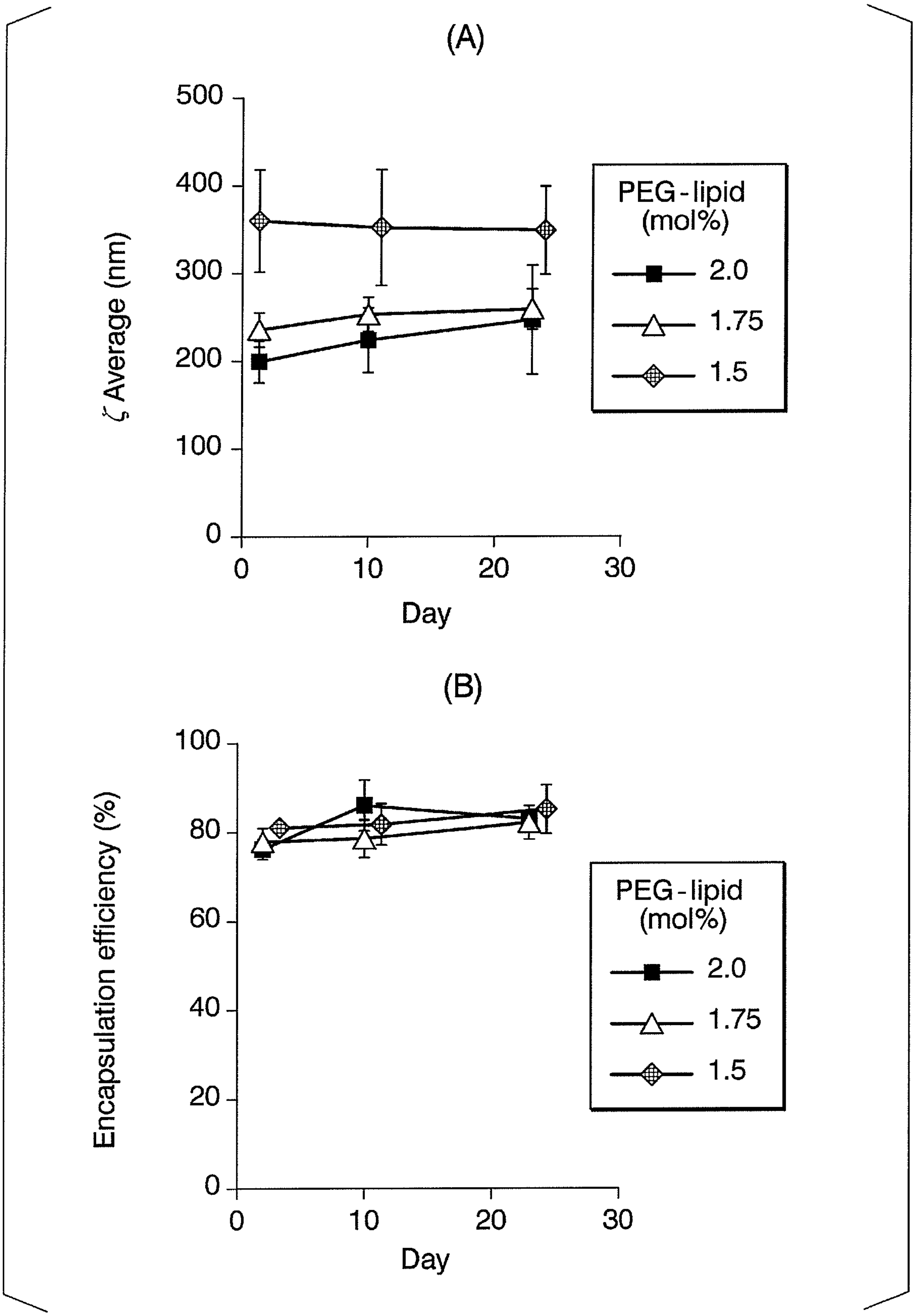
(A)
500
400
300
200
100
0
ζ Average (nm)
0
10
20
30
Day
PEG - lipid (mol%)
2.0
1.75
1.5
(B)
100
80
60
40
20
0
Encapsulation efficiency (%)
0
10
20
30
Day
PEG - lipid (mol%)
2.0
1.75
1.5

[FIG. 12]

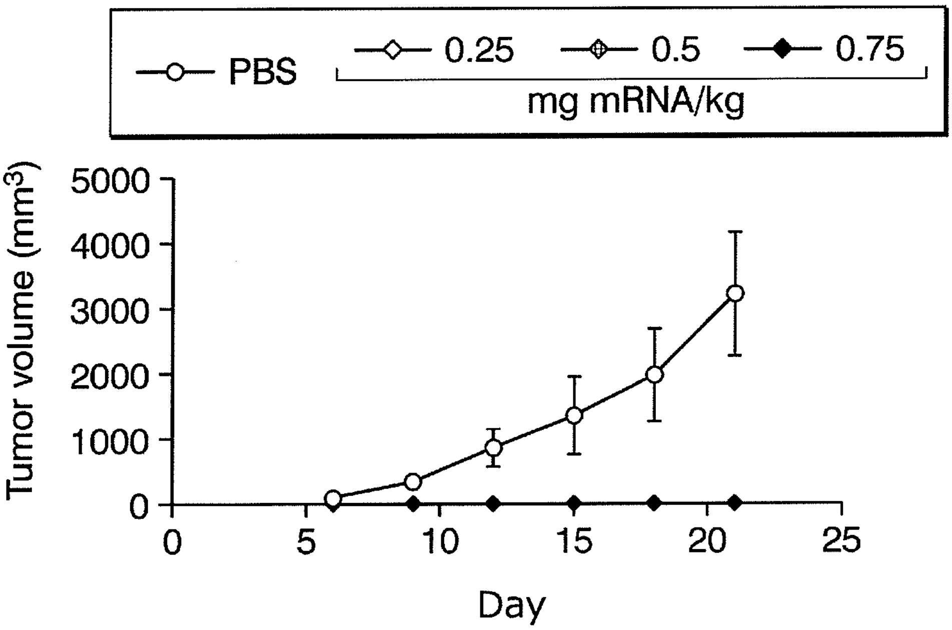
PBS
0.25
0.5
0.75
mg mRNA/kg
Tumor volume (mm3)
5000
4000
3000
2000
1000
0
0
5
10
15
20
25
Day

[FIG. 13]

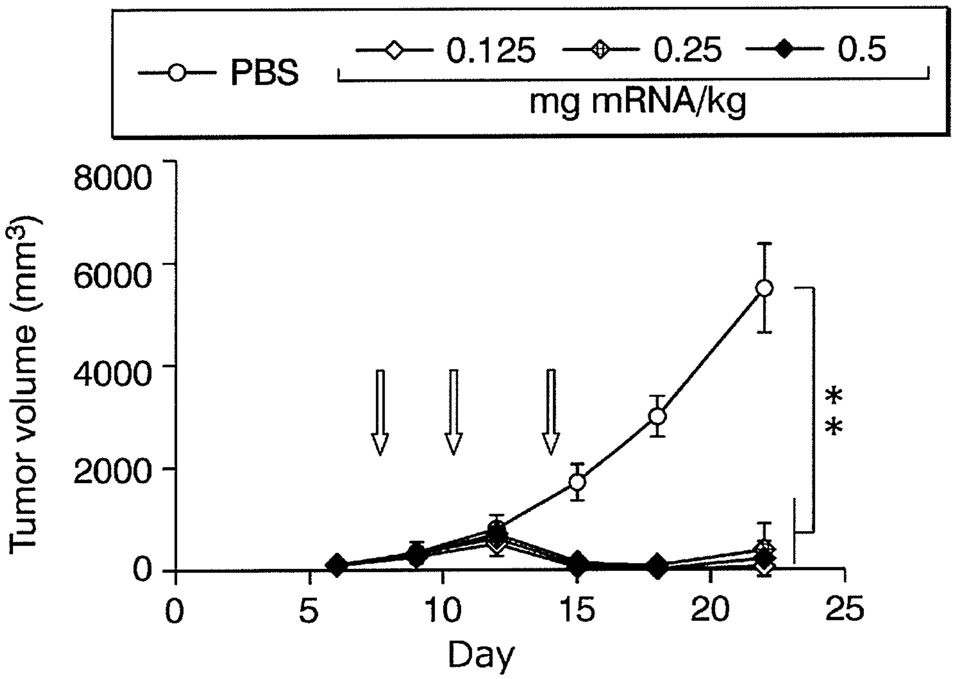
PBS
0.125
0.25
0.5
mg mRNA/kg
Tumor volume (mm3)
8000
6000
4000
2000
0
0
5
10
15
20
25
Day
**

[FIG. 14]

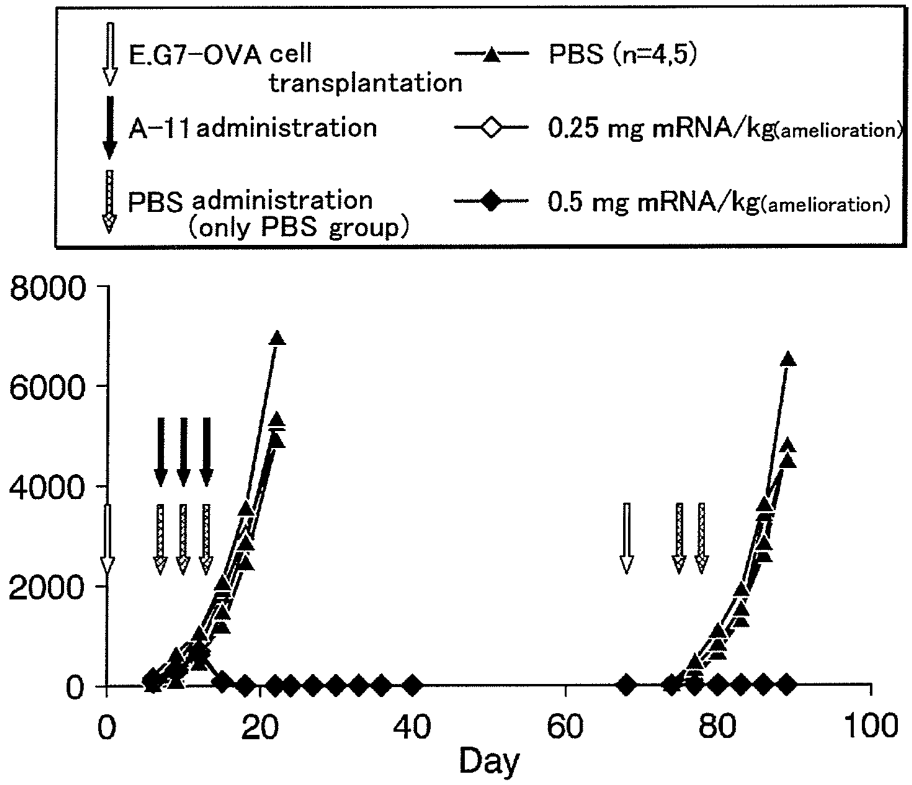
E.G7-OVA cell transplantation
A-11 administration
PBS administration (only PBS group)
PBS (n=4,5)
0.25 mg mRNA/kg(amelioration)
0.5 mg mRNA/kg(amelioration)
8000
6000
4000
2000
0
0
20
40
60
80
100
Day

[FIG. 15]

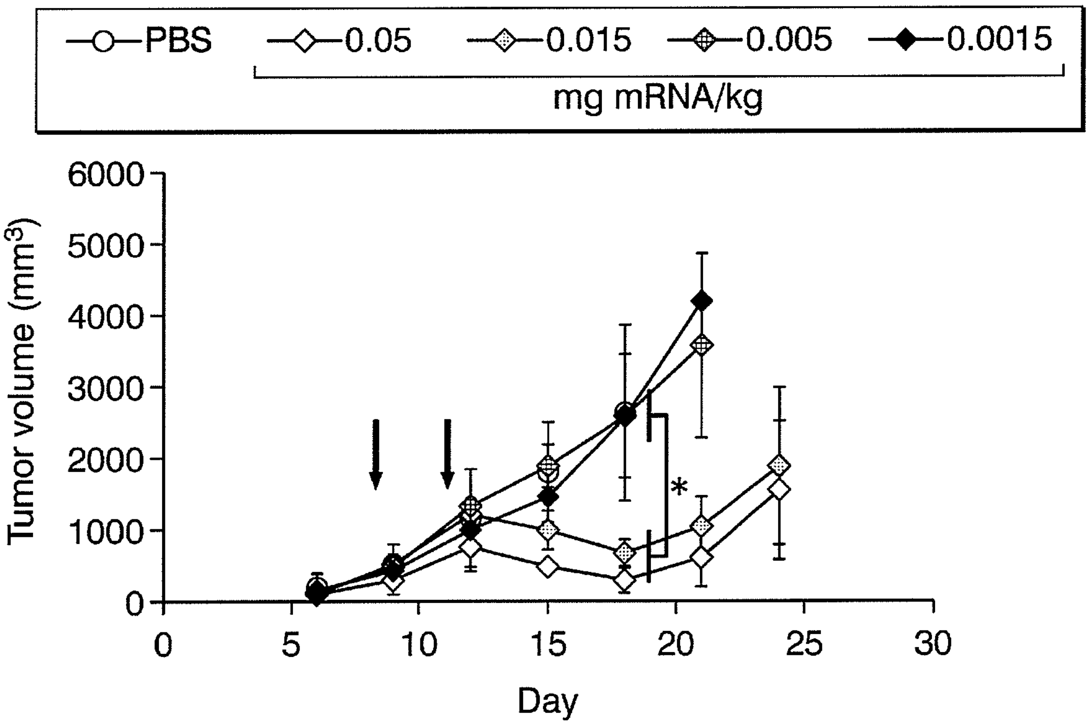
PBS
0.05
0.015
0.005
0.0015
mg mRNA/kg
Tumor volume ($mm^3$)
6000
5000
4000
3000
2000
1000
0
0
5
10
15
20
25
30
Day
*

[FIG. 16]

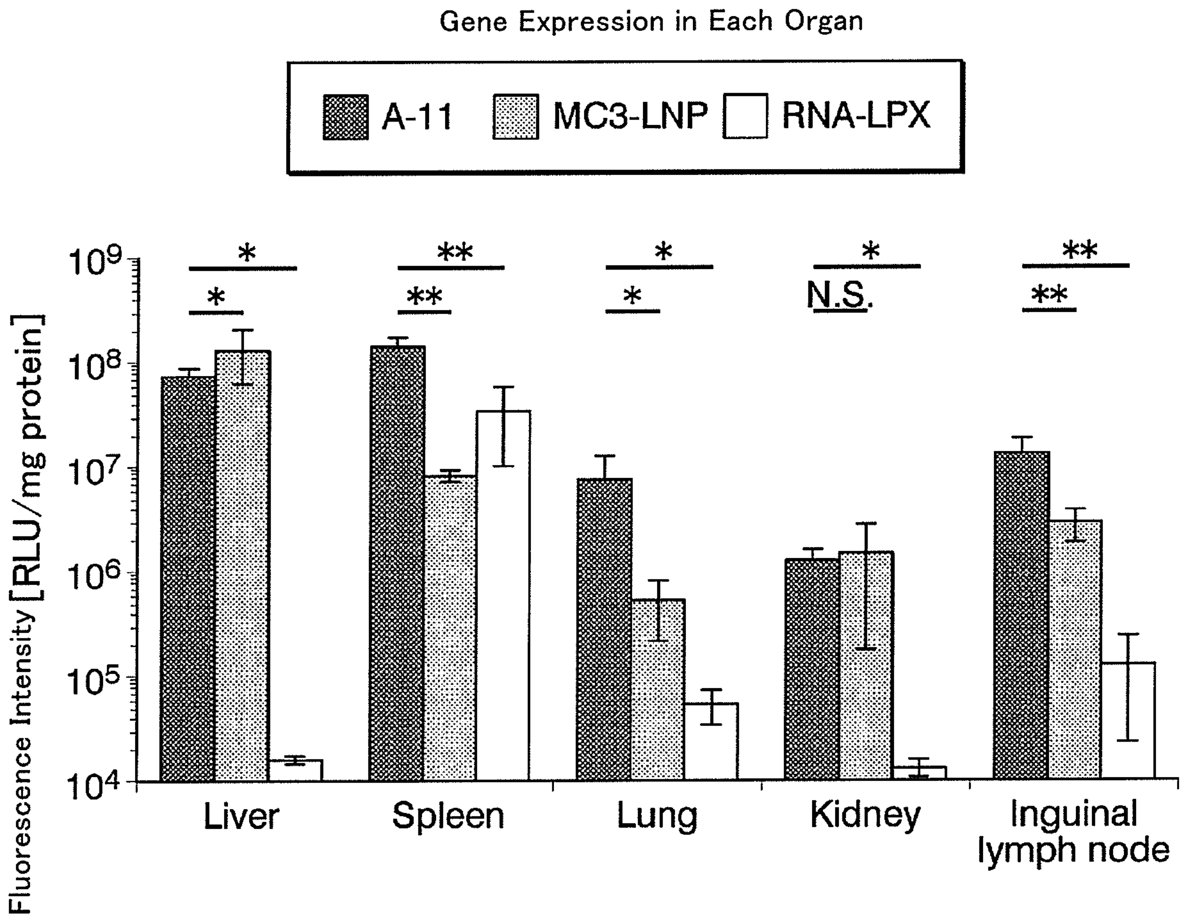
Gene Expression in Each Organ
A-11
MC3-LNP
RNA-LPX
Fluorescence Intensity [RLU/mg protein]
10^9
10^8
10^7
10^6
10^5
10^4
*
*
**
**
*
*
*
N.S.
**
**
Liver
Spleen
Lung
Kidney
Inguinal lymph node

[FIG. 17]

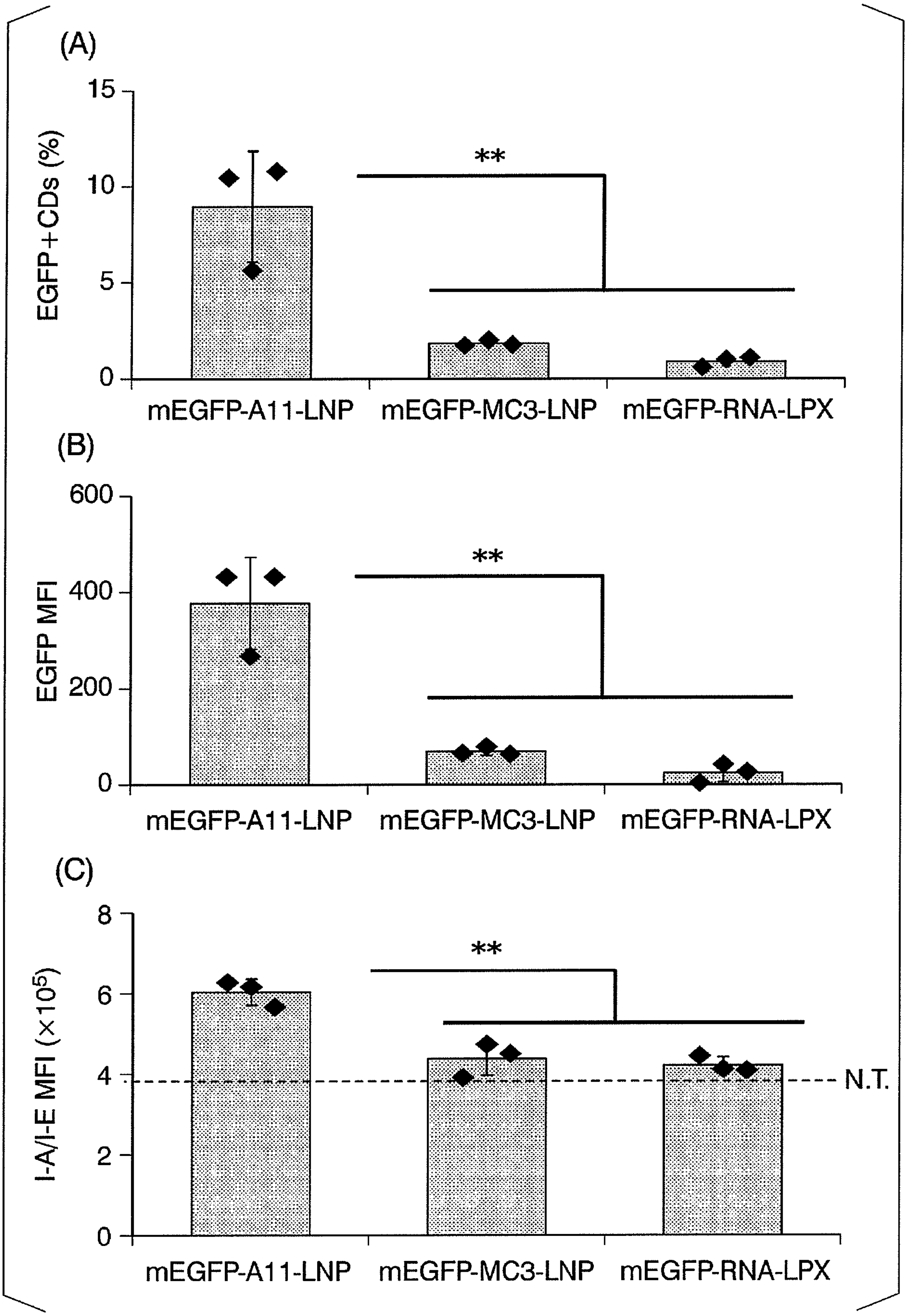

(A)
15
10
5
0
EGFP+CDs (%)
**
mEGFP-A11-LNP
mEGFP-MC3-LNP
mEGFP-RNA-LPX
(B)
600
400
200
0
EGFP MFI
**
mEGFP-A11-LNP
mEGFP-MC3-LNP
mEGFP-RNA-LPX
(C)
8
6
4
2
0
I-A/I-E MFI (×10^5)
**
N.T.
mEGFP-A11-LNP
mEGFP-MC3-LNP
mEGFP-RNA-LPX

[FIG. 18]

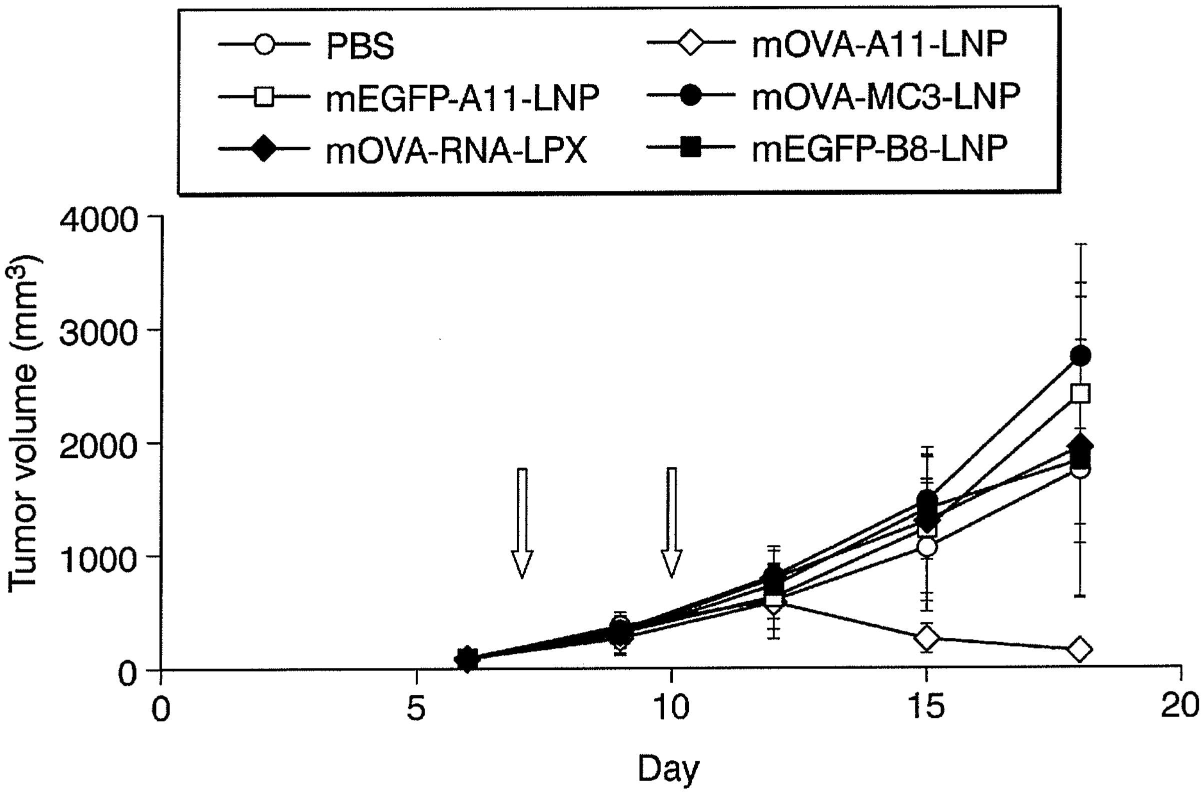
PBS
mEGFP-A11-LNP
mOVA-RNA-LPX
mOVA-A11-LNP
mOVA-MC3-LNP
mEGFP-B8-LNP
Tumor volume ($mm^3$)
4000
3000
2000
1000
0
0
5
10
15
20
Day

## INTERNATIONAL SEARCH REPORT

International application No. **PCT/JP2022/020849**

### A. CLASSIFICATION OF SUBJECT MATTER

***A61K 47/16***(2006.01)i; ***A61K 9/10***(2006.01)i; ***A61K 9/127***(2006.01)i; ***A61K 31/7105***(2006.01)i; ***A61K 31/713***(2006.01)i; ***A61K 47/02***(2006.01)i; ***A61K 47/24***(2006.01)i; ***A61K 47/28***(2006.01)i; ***A61K 47/44***(2017.01)i; ***A61K 48/00***(2006.01)i; ***A61P 35/00***(2006.01)i; ***A61P 37/04***(2006.01)i

FI: A61K47/16; A61K9/127; A61K31/713; A61K47/02; A61K47/44; A61K48/00; A61P35/00; A61P37/04; A61K47/24; A61K9/10; A61K47/28; A61K31/7105

According to International Patent Classification (IPC) or to both national classification and IPC

### B. FIELDS SEARCHED

Minimum documentation searched (classification system followed by classification symbols)

A61K47/16; A61K9/10; A61K9/127; A61K31/7105; A61K31/713; A61K47/02; A61K47/24; A61K47/28; A61K47/44; A61K48/00; A61P35/00; A61P37/04

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

JSTPlus/JMEDPlus/JST7580 (JDreamIII); CAplus/MEDLINE/EMBASE/BIOSIS (STN)

### C. DOCUMENTS CONSIDERED TO BE RELEVANT

| Category* | Citation of document, with indication, where appropriate, of the relevant passages | Relevant to claim No. |
|---|---|---|
| X | WO 2020/262150 A1 (NATIONAL UNIVERSITY CORP. HOKKAIDO UNIVERSITY) 30 December 2020 (2020-12-30) examples 1-9, tables 3-4, 7, paragraphs [0031]-[0035] | 1-2, 4-12 |
| Y | | 3, 13-16 |
| Y | WO 2018/230710 A1 (NATIONAL UNIVERSITY CORP. HOKKAIDO UNIVERSITY) 20 December 2018 (2018-12-20) claims, examples 1-10, paragraphs [0015]-[0016], [0089] | 3, 13-16 |
| Y | WO 2018/190423 A1 (NATIONAL UNIVERSITY CORP. HOKKAIDO UNIVERSITY) 18 October 2018 (2018-10-18) paragraphs [0002], [0046]-[0047], claims, examples, figures | 13-16 |
| Y | JP 2009-507876 A (NOVOSOM AG) 26 February 2009 (2009-02-26) paragraphs [0082]-[0088] | 13-16 |

☑ Further documents are listed in the continuation of Box C. ☑ See patent family annex.

* Special categories of cited documents:
- "A" document defining the general state of the art which is not considered to be of particular relevance
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **14 June 2022** | **28 June 2022** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (January 2015)

**INTERNATIONAL SEARCH REPORT**

International application No.
**PCT/JP2022/020849**

**C. DOCUMENTS CONSIDERED TO BE RELEVANT**

| Category* | Citation of document, with indication, where appropriate, of the relevant passages | Relevant to claim No. |
|---|---|---|
| Y | 山本浩充ほか, 粒子物性制御による微粒子ドラッグ デリバリーシ ステムの機能設計, Drug Delivery System. 2002, vol. 17-4, pages 321-329, (YAMAMOTO, Hiromitsu et al. Functional design of microparticulate drug delivery systems by controlling particle physical properties.) page 321, right column, lines 1-4, page 322, right column, lines 3-9 | 13-16 |
| A | JP 2019-151589 A (NATIONAL UNIVERSITY CORP. HOKKAIDO UNIVERSITY) 12 September 2019 (2019-09-12) whole document | 1-16 |
| A | JP 2013-245190 A (BIOMEDCORE INC.) 09 December 2013 (2013-12-09) whole document | 1-16 |
| A | 佐藤悠介ほか, 新規pH応答性カチオン性脂質を用いた効率的なshort interference RNA(siRNA)デリバリーシ ステムの開発, YAKUGAKU ZASSHI. 2012, vol. 132 (12), pages 1355-1363, (SATO, Yusuke et al. Development of an Efficient Short Interference RNA (siRNA) Delivery System with a New pH-Sensitive Cationic Lipid.) whole document | 1-16 |

Form PCT/ISA/210 (second sheet) (January 2015)

**INTERNATIONAL SEARCH REPORT**
**Information on patent family members**

International application No.
**PCT/JP2022/020849**

| Patent document cited in search report | Publication date (day/month/year) | Patent family member(s) | Publication date (day/month/year) |
|---|---|---|---|
| WO 2020/262150 A1 | 30 December 2020 | (Family: none) | |
| WO 2018/230710 A1 | 20 December 2018 | US 2020/0129431 A1<br>claims, examples 1-10, paragraphs [0046]-[0047], [0147]<br>EP 3640237 A1<br>CN 110740985 A<br>KR 10-2020-0018782 A | |
| WO 2018/190423 A1 | 18 October 2018 | US 2021/0129103 A1<br>paragraphs [0002], [0080]-[0081], claims, examples, figures<br>EP 3610943 A1<br>CN 110520214 A | |
| JP 2009-507876 A | 26 February 2009 | US 2007/0104775 A1<br>paragraphs [0117]-[0122]<br>WO 2006/048329 A1 | |
| JP 2019-151589 A | 12 September 2019 | (Family: none) | |
| JP 2013-245190 A | 09 December 2013 | (Family: none) | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2018230710 A
- WO 2018190423 A
- WO 2007102481 A


### Non-patent literature cited in the description


- **PARDI et al.** *NATURE REVIEWS DRUG DISCOVERY,* 2018, vol. 17, 261-279
- **KRANZ et al.** *Nature,* 2016, vol. 534, 396-401
- **SAHIN et al.** *Nature,* 2020, vol. 585, 107-112
- **POLACK et al.** *The New ENGLAND JOURNAL of MEDICINE,* 2020, vol. 383 (27), 2603-2615
- **SATO et al.** *Journal of Controlled Release,* 2019, vol. 295, 140-152
- **STROOCK et al.** *Science,* 2002, vol. 295, 647-651
- **HAJJ et al.** *Nano Letters,* 2020, vol. 20, 5167-5175